(19) Europäisches Patentamt
European Patent Office
Office européen des brevets

(11) **EP 2 458 368 B1**

(12) **EUROPÄISCHE PATENTSCHRIFT**

(45) Veröffentlichungstag und Bekanntmachung des Hinweises auf die Patenterteilung:
**23.07.2014 Patentblatt 2014/30**

(51) Int Cl.:
**G01N 21/17** *(2006.01)* **A61B 5/145** *(2006.01)*

(21) Anmeldenummer: **10192469.4**

(22) Anmeldetag: **24.11.2010**

(54) **Erfassungsvorrichtung zum Erfassen eines Blutbildparameters**

Recording device for recording a blood count parameter

Dispositif de détection pour la détection d'un paramètre d'hémogramme

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.05.2012 Patentblatt 2012/22**

(73) Patentinhaber:
• **eesy-id GmbH**
  **82166 Gräfelfing (DE)**
• **Friedrich-Alexander-Universität Erlangen-Nürnberg**
  **91054 Erlangen (DE)**

(72) Erfinder: **Fischer, Georg**
**90425 Nürnberg (DE)**

(74) Vertreter: **Klinski, Robert et al**
**PATENTSHIP**
**Patentanwaltskanzlei**
**Elsenheimerstraße 65**
**80687 München (DE)**

(56) Entgegenhaltungen:
**WO-A1-2010/131029    US-A1- 2006 025 664**
**US-A1- 2009 275 814**

**Beschreibung**

Die vorliegende Erfindung betrifft das Gebiet der Erfassung einer Konzentration eines Blutbestandteiles, beispielsweise Zucker in durch ein Blutgefäß fließenden Blut.

Zur Bestimmung eines Blutbildparameters wie beispielsweise einer Konzentration eines Blutbestandteils kann invasiv Blut entnommen werden. Der Blutbildparameter kann dann unter Verwendung des entnommenen Bluts anhand standardisierter Teststreifen bestimmt werden, deren elektrische Widerstandswerte von der Konzentration des Blutbestandteiles, beispielsweise Blutzuckers, abhängen. Der jeweilige elektrische Widerstandswert kann beispielsweise unter Verwendung eines Blutzuckermessgerätes, das eine Gleichstrom-Widerstandsmessung zur Erfassung eines elektrischen Widerstandswertes eines Teststreifens durchführt, erfasst werden. Der Widerstandswert kann anhand eines an sich bekannten Zusammenhanges zwischen einer Blutzuckerkonzentration und einem Widerstandswert in eine Blutzuckerkonzentration umgerechnet werden. Zur Erzielung einer hohen Erfassungsgenauigkeit ist jeder Teststreifen mit Kalibrierdaten, beispielsweise mit einem Referenzwiderstandswert oder mit einer entsprechenden Codierung, versehen, wodurch Variationen von Eigenschaften der Teststreifen ausgeglichen werden können. Nachteilig an invasiven Verfahren ist jedoch die Notwendigkeit der Blutentnahme und somit einer Verletzung eines Patienten. Außerdem ist eine kontinuierliche Erfassung einer Konzentration eines Blutbestandteils, um beispielsweise dessen Tagesgangkurve zu ermitteln, aufwändig. Darüber hinaus kann mittels des invasiven Verfahrens eine Zeitverzögerung zwischen einer Nahrungsaufnahme und beispielsweise einem Anstieg des Blutzuckers nicht genau erfasst werden. Ferner kann insbesondere bei einer geringen Konzentration des Blutzuckers in Blut ein Zeitpunkt der Insulinabgabe an den Patienten nicht genau bestimmt werden.

Zur nichtinvasiven Bestimmung eines Blutbildparameters wie beispielsweise einer Stoffkonzentration oder einer Stoffzusammensetzung in Blut können mikrowellenspektroskopische Verfahren eingesetzt werden. Die Mikrowellenspektroskopie zur Erfassung von Blutbildparametern basiert auf einer Einkopplung eines Mikrowellensignals in ein blutdurchströmtes Gewebe und einer Erfassung einer frequenzabhängigen Absorption eingekoppelter Mikrowellenenergie.

Die Druckschrift US 2009/027814 A1 beschreibt ein System und ein Verfahren zum Messen eines Bestandteils einer Probe. Zu diesem Zweck umfasst das System einen Sender, der eine elektromagnetische Welle mit einer variablen Frequenz in Richtung der Probe ausgibt. Die von der Probe reflektierte Welle wird von einem Detektor empfangen. Die elektrische Welle dringt an einer unbestimmten Stelle in das Gewebe ein, so dass die Messstrecke nicht eindeutig definiert und reproduzierbar ist.

In der Druckschrift von Andreas Caduff et al., "Non-invasive glucose monitoring in patients with Type 1 diabetes: A multi-sensor system combining sensors for dielectric and optical characterization of skin", Biosensors and Bioelectronics 24 (2009) 2778-2784, ist eine Mehrelektrodenanordnung zur mikrowellenbasierten Bestimmung eines Blutbildparameters beschrieben. Die Mehrelektrodenanordnung umfasst mehrere Elektrodenpaare mit unterschiedlichen Elektrodenabständen, durch welche unterschiedliche Eindringtiefen von Mikrowellensignalen realisiert werden können. Die Erfassung des Blutbildparameters erfolgt mittels einer Impedanzmessung, also mittels einer Eintormessung, und ist daher fehleranfällig bei etwaigen Impedanzfehlanpassungen. Aufgrund von unterschiedlichen Eindringtiefen ist mitunter keine Unterscheidung zwischen kapillarem und venösem Blut möglich, was die Messergebnisse verfälschen kann. Eine Messung eines Blutbildparameters an venösem Blut ist im Allgemeinen genauer als eine Messung des Blutbildparameters an kapillarem Blut, weil beispielsweise Blutzuckeränderungen in kapillarem Blut verzögert gegenüber venösem Blut sind.

In den Druckschriften von Buford Randal Jean et al., "A microwave frequency sensor for non-invasive blood-glucose measurement, SAS 2008 - IEEE Sensors Applications Symposium, Atlanta, GA, February 12 - 14, 2008, and M. McClung, Calibration methodology for a microwave non-invasive glucose sensor, Master Thesis, Baylor University, Mai 2008, ist eine weitere Elektrodenanordnung zur Bestimmung einer Blutzuckerkonzentration beschrieben. Dabei wird ausgenutzt, dass die dielektrischen Eigenschaften von Blut von einem Blutzuckergehalt abhängen. Durch Anpressen eines Daumens auf den Mikrowellensensor wird eine Änderung der Dielektrizitätszahl des Daumens durch eine Verstimmung eines Resonators gemessen. Durch das Anpressen des Daumes wird jedoch Blut verdrängt, was zu einer Verfälschung der Messergebnisse führen kann. Des Weiteren können die Messungen nicht kontinuierlich durchgeführt werden. Die Auswertung der Messdaten zur Bestimmung des Blutzuckergehalts hängt zudem von dem jeweiligen Patienten ab und ist daher bei anderen Patienten nicht reproduzierbar. Darüber hinaus ist mit diesem Verfahren die Eindringtiefe der Mikrowellenleistung nicht steuerbar, so dass eine Unterscheidung zwischen kapillarem und venösem Blut nicht möglich ist. Ferner wird die Änderung der Dielektrizitätszahl auf der Basis einer Eintormessung durchgeführt, welche anfällig bezüglich Fehlanpassungen ist.

Es ist die Aufgabe der vorliegenden Erfindung, ein effizientes Konzept zur mikrowellenbasierten, nicht invasiven Bestimmung eines Blutbildparameters, insbesondere einer Konzentration von Blutzucker, in einem durch ein Blutgefäß fließenden Blut zu schaffen.

Diese Aufgabe wird durch die Merkmale der unabhängigen Ansprüche gelöst. Vorteilhafte Weiterbildungsformen sind Gegenstand der abhängigen Ansprüche.

Die Erfindung basiert auf der Erkenntnis, dass ein Blutbildparameter durch eine Erfassung einer Relaxationszeitkonstante eines Blutbestandteils ermittelt werden kann. Ist der zu bestimmende Blutbildparameter beispielsweise eine Konzentration von Blutzucker in Blut, so ist eine Relaxationszeitkonstante einer Zucker enthaltenden Wasserlösung ein Maß für die Konzentration des Blutzuckers, d.h. für den Blutzuckerspiegel.

Die Erfindung basiert auf der weiteren Erkenntnis, dass die Relaxationszeitkonstante des Blutbestandteils durch Messungen von in das Blutgefäß eingekoppelten Mikrowellensignalen bestimmt werden kann. Dabei werden Verlustgrößen der eingekoppelten Mikrowellensignale erfasst. Die Verlustgrößen werden beispielsweise durch den frequenzabhängigen Verlauf der komplexen Dielektrizitätszahl repräsentiert.

Die Erfindung basiert auf der weiteren Erkenntnis, dass ein Blutgefäß wie beispielsweise eine Vene oder eine Arterie, das dieses Blutgefäß umgebende Fettgewebe sowie die darüber liegende Hautschicht als ein dielektrisches Wellenleitersystem aufgefasst werden kann. Bei einer Anregung eines derartigen dielektrischen Wellenleitersystems können daher unterschiedliche Moden bzw. Wellentypen angeregt werden, beispielsweise transversal-elektromagnetische (TEM) Wellen oder transversal-elektrische (TE) Wellen oder transversal-magnetische (TM) Wellen oder eine HE-Welle. Bei einer TE-Mode existiert eine in Ausbreitungsrichtung weisende, von Null verschiedene Komponente des Magnetfeldes. Bei einer TM-Mode existiert hingegen eine in Modenausbreitungsrichtung weisende, von Null verschiedene Komponente eines elektrischen Feldes. In Abhängigkeit von einer Hochfrequenzanregung können daher in einem das Blutgefäß und die Hautschicht umfassenden dielektrischen Wellenleitersystem unterschiedliche, auch in Blutflussrichtung ausbreitungsfähige Moden angeregt werden, wodurch eine genaue Erfassung eines Blutbildparameters möglich ist.

Das Blutgefäß, in welche die Sendesignale eingekoppelt und die Empfangssignale ausgekoppelt werden, wird als dielektrischer Wellenleiter interpretiert. Die Sendesignale sind insbesondere als Mikrowellensignale ausgebildet. Durch die Verwendung der Mikrowellensignale wird eine robuste Messmethodik ermöglicht.

Mittels der bestimmten Relaxationszeitkonstanten ($\tau$) kann zumindest ein Blutbildparameter, z.B. die Glucosekonzentration im Blut, ermittelt werden. Der Blutbildparameter kann - wie die Relaxationszeitkonstante ($\tau$) des Blutbestandteils - kontinuierlich ermittelt werden. Beispielsweise für die Glucosekonzentration als Blutbildparameter ergibt sich hierdurch ein Vorteil gegenüber konventionellen Lösungen: Es wird eine Bestimmung der Verzögerungszeit zwischen der Nahrungsaufnahme des Patienten und dem Blutzuckeranstieg ermöglicht. Auf Variationen im Tagesablauf des Patienten kann folglich schneller reagiert werden. Bei einer sich abzeichnenden Überzuckerung oder Unterzuckerung kann sofort ein Alarm ausgelöst werden. Auch eine telemedizinische Anbindung über eine Kommunikationsschnittstelle ist möglich.

Durch die Verwendung des ersten Sendesignals, des zweiten Sendesignals und potenziell weiterer Sendesignale ist eine breitbandige Messung oder Ermittlung hinsichtlich der Verlustgrößen möglich.

Gemäß einem Aspekt der Erfindung wird eine Erfassungsvorrichtung zum Erfassen eines Blutbildparameters eines Blutbestandteils von Blut in einem Blutgefäß vorgeschlagen, welche einen Sender, einen Empfänger, einen Verlustdetektor und einen Prozessor aufweist. Der Sender ist dazu eingerichtet, in das Blutgefäß ein erstes Sendesignal mit einer ersten Frequenz und ein zweites Sendesignal mit einer zweiten Frequenz einzukoppeln. Der Empfänger ist dazu eingerichtet, ein erstes Empfangssignal bei der ersten Frequenz und ein zweites Empfangssignal bei der zweiten Frequenz zu empfangen. Der Verlustdetektor ist ausgebildet, eine erste Verlustgröße auf der Basis des ersten Sendesignals und des ersten Empfangssignals zu ermitteln. Weiter ist der Verlustdetektor ausgebildet, eine zweite Verlustgröße auf der Basis des zweiten Sendesignals und des zweiten Empfangssignals zu ermitteln. Der Prozessor ist ausgebildet, eine Relaxationszeitkonstante ($\tau$) des Blutbestandteils in Abhängigkeit von der Frequenz mit der größeren Verlustgröße zu bestimmen.

Insbesondere ist der Prozessor dazu eingerichtet, die Relaxationszeitkonstante ($\tau$) des Blutbestandteils in Abhängigkeit von der ersten Frequenz zu bestimmen, wenn die erste Verlustgröße nicht kleiner als die zweite Verlustgröße ist, oder die Relaxationszeitkonstante ($\tau$) des Blutbestandteils in Abhängigkeit von der zweiten Frequenz zu bestimmen, wenn die zweite Verlustgröße nicht kleiner als die erste Verlustgröße ist.

Gemäß einer Ausführungsform ist der Prozessor dazu ausgebildet, zumindest einen Blutbildparameter in Abhängigkeit der bestimmten Relaxationszeitkonstanten ($\tau$) zu ermitteln.

Gemäß einer Ausführungsform ist der Prozessor dazu ausgebildet, zumindest einen Blutbildparameter in Abhängigkeit der bestimmten Relaxationszeitkonstanten ($\tau$) mittels eines vorgegebenen Zusammenhangs zwischen der Konzentration des Blutbildparameters und der Relaxationszeitkonstanten ($\tau$) zu ermitteln.

Gemäß einer Ausführungsform umfasst der vorgegebene Zusammenhang eine Abbildung der Konzentration des Blutbildparameters auf die Relaxationszeitkonstante ($\tau$).

Gemäß einer Ausführungsform umfasst die Erfassungsvorrichtung eine Nachschlagtabelle, mittels welcher der vorgegebene Zusammenhang zwischen der Konzentration des Blutbildparameters und der Relaxationszeitkonstanten ($\tau$) abgebildet wird.

Gemäß einer Ausführungsform ist der zumindest eine Blutbildparameter eine Glucosekonzentration in dem Blut, eine Laktatkonzentration in dem Blut oder eine Sauerstoffkonzentration in dem Blut umfasst.

Gemäß einer Ausführungsform ist der Verlustdetektor ausgebildet, die erste Verlustgröße und die zweite Verlustgröße durch eine Zweitormessung zu bestimmen.

Vorteilhafterweise liefert die Zweitormessung ein sichereres Messergebnis als eine herkömmliche Eintormessung.

Gemäß einer Ausführungsform umfasst der Verlustdetektor einen Netzwerkanalysator oder einen Leistungsdetektor.

Gemäß einer Ausführungsform ist der Verlustdetektor ausgebildet, zur Bestimmung der ersten Verlustgröße und der zweiten Verlustgröße jeweils einen Vorwärtstransmissionsfaktor $S_{21}$ und/oder einen Eingangsreflexionsfaktor $S_{11}$ zu bestimmen.

Gemäß einer Ausführungsform ist der Verlustdetektor ausgebildet, die erste Verlustgröße und die zweite Verlustgröße jeweils auf der Basis der folgenden Formel zu bestimmen:

$$P_{Verlust} = 1 - \left|S_{11}\right|^2 - \left|S_{21}\right|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

Gemäß einer Ausführungsform ist der Prozessor dazu ausgebildet ist, die Relaxationszeitkonstante ($\tau$) auf der Basis der folgenden Formel zu bestimmen:

$$\tau = \frac{1}{2\pi f_A},$$

wobei $f_A$ die Frequenz bezeichnet, bei welcher die ermittelte Verlustgröße größer ist.

Gemäß einer Ausführungsform ist der Verlustdetektor dazu ausgebildet, die komplexe Dielektrizitätszahl ($\varepsilon''$) bei der jeweiligen Frequenz zur Bestimmung der jeweiligen Verlustgröße zu ermitteln.

Dabei wird insbesondere der Imaginärteil der komplexen Dielektriziätskonstanten oder Dielektrizitätszahl ausgewertet. Insbesondere werden die Frequenzen betrachtet, bei denen der Imaginärteil der komplexen Dielektrizitätszahl ein lokales Maximum hat. Dadurch wird eine Separation verschiedener polarer Effekte durch Betrachtung des Imaginärteils der komplexen Dielektrizitätskonstanten ermöglicht, welche die frequenzabhängigen Verluste darstellt.

Gemäß einer Ausführungsform ist der Prozessor dazu ausgebildet, die Frequenz zu bestimmen, bei welcher der Imaginärteil der komplexen Dielektrizitätszahl ($\varepsilon''$) maximal ist, und die Relaxationszeitkonstante ($\tau$) in Abhängigkeit der bestimmten Frequenz zu ermitteln.

Gemäß einer Ausführungsform ist der Sender dazu ausgebildet, zumindest ein Sendesignal mit einer Vielzahl von Frequenzen in das Blutgefäß einzukoppeln. Dabei ist der Empfänger dazu ausgebildet, zumindest ein Empfangssignal mit der Vielzahl von Frequenzen zu empfangen. Weiter ist der Prozessor dazu ausgebildet, die Frequenz zu bestimmen, bei welcher die komplexe Dielektrizitätszahl ($\varepsilon''$) maximal ist, und die Relaxationszeitkonstante ($\tau$) in Abhängigkeit der bestimmten Frequenz zu ermitteln.

Gemäß einer Ausführungsform hat der Sender zum Einkoppeln des ersten Sendesignals oder des zweiten Sendesignals zumindest eine Sendeantenne, insbesondere eine Dipol-Antenne, eine Rahmen-Antenne oder eine Patch-Antenne. Gemäß dieser bevorzugten Ausführungsform hat der Empfänger zum Empfangen des ersten Empfangssignals und des zweiten Empfangssignals zumindest eine Empfangsantenne, insbesondere eine Dipol-Antenne oder eine Rahmen-Antenne, welche von der Sendeantenne beabstandet ist.

Gemäß einer Ausführungsform ist der Sender ausgebildet, das erste Sendesignal oder das zweite Sendesignal als eine transversal-elektrische (TE) Welle oder als eine transversal-magnetische (TM) Welle in das Blutgefäß einzukoppeln, insbesondere longitudinal oder transversal bezüglich einer Blutflußrichtung einzukoppeln.

Gemäß einer Ausführungsform ist der Sender ausgebildet, das erste Sendesignal und das zweite Sendesignal sukzessive, insbesondere mittels eines durchstimmbaren Oszillators, oder gleichzeitig, insbesondere mittels des das erste Sendesignal und das zweite Sendesignal umfassenden Breitbandsignals, in das Blutgefäß einzukoppeln.

Gemäß einer Ausführungsform ist das Sendesignal ein breitrandiges HochfrequenzSignal oder ein Sweep-Signal.

Gemäß einer Ausführungsform ist das Sendesignal als ein Mikrowellensignal ausgebildet.

Gemäß einer Ausführungsform ist das Blutgefäß eine Ader oder eine Vene.

Gemäß einer Ausführungsform koppelt der Sender das Sendesignal mit einer Leistung von 0,1 bis 1,0 mW in das Blutgefäß ein.

Gemäß einer Ausführungsform liegen die erste Frequenz und die zweite Frequenz jeweils in einem Frequenzbereich zwischen 1 GHz und 15 GHz.

Gemäß einer Ausführungsform umfasst die Erfassungsvorrichtung einen Sender mit einer Anzahl von Sendeantennen zum Aussenden von zumindest einem Sendesignal, einen Empfänger mit einer Anzahl von Empfangsantennen zum Empfangen von zumindest einem Empfangssignal, den Prozessor und den Verlustdetektor. Dabei ist der Prozessor dazu ausgebildet, eine erste Erfassungskonfiguration umfassend eine Sendeantenne der Anzahl der Sendeantennen und eine Empfangsantenne der Anzahl der Empfangsantennen auszuwählen, und eine zweite Erfassungskonfiguration umfassend eine Sendeantenne der Anzahl der Sendeantennen und eine Empfangsantenne der Anzahl von Empfangsantennen auszuwählen. Ferner ist der Verlustdetektor dazu ausgebildet, bei Auswahl der ersten Erfassungskonfiguration zum Aussenden eines Sendesignals eine erste Verlustgröße auf der Basis des Sendesignals und eines Empfangssignals zu erfassen, und bei Auswahl der zweiten Erfassungskonfiguration zum Aussenden eines Sendesignals eine zweite Verlustgröße auf der Basis des Sendesignals und eines Empfangssignals zu erfassen. Weiterhin ist der Prozessor dazu ausgebildet, die Erfassungskonfiguration mit der geringeren Verlustgröße zum Erfassen des Blutbildparameters auszuwählen.

Der Sender ist beispielsweise als ein Sender mit einem breitbandigen Pseudorauschsignal ausgebildet, zum Beispiel als ein M-Sequenz Radar.

Insbesondere ist der Sender bei Auswahl der ersten Erfassungskonfiguration dazu ausgebildet, das Sendesignal mittels der Sendeantenne der ersten Erfassungskonfiguration auszusenden. Der Empfänger ist bei Auswahl der ersten Erfassungskonfiguration dazu ausgebildet, das Empfangssignal mittels der Empfangsantenne der ersten Erfassungskonfiguration zu empfangen. Des Weiteren ist der Sender bei Auswahl der zweiten Erfassungskonfiguration dazu ausgebildet, das Sendesignal mittels der Sendeantenne der zweiten Erfassungskonfiguration auszusenden, wobei der Empfänger bei Auswahl der zweiten Erfassungskonfiguration ausgebildet ist, das Empfangssignal mittels der Empfangsantenne der zweiten Erfassungskonfiguration zu empfangen. Dabei ist der Verlustdetektor ausgebildet, die erste Verlustgröße auf der Basis des Sendesignals und des Empfangssignals der ersten Erfassungskonfiguration zu erfassen, und die zweite Verlustgröße auf der Basis des Sendesignals und des Empfangssignals der zweiten Erfassungskonfiguration zu erfassen.

Gemäß einer Ausführungsform sind die erste Verlustgröße eine Absorptionslinie einer Wasserlösung mit einem Blutbestandteil bei der ersten Frequenz und die zweite Verlustgröße eine Absorptionslinie der Wasserlösung bei der zweiten Frequenz.

Gemäß einer Ausführungsform definieren die erste Verlustgröße und die zweite Verlustgröße einen frequenzabhängigen Verlauf von Absorptionslinien einer Wasserlösung mit einem Blutbestandteil.

Gemäß einer Ausführungsform ist die erste Verlustgröße ein Absorptionsminimum oder ein Absorptionsmaximum in einem die erste Frequenz umfassenden ersten Frequenzbereich, wobei die zweite Verlustgröße ein Absorptionsminimum oder ein Absorptionsmaximum in einem die zweite Frequenz umfassenden zweiten Frequenzbereich ist.

Gemäß einer Ausführungsform betrifft die Erfindung eine Erfassungsvorrichtung zum Erfassen eines Blutbildparameters von Blut in einem Blutgefäß, mit einem Sender mit einer Anzahl von Sendeantennen zum Aussenden von zumindest einem Sendesignal, einem Empfänger mit einer Anzahl von Empfangsantennen zum Empfangen von zumindest einem Empfangssignal, einem Prozessor, welcher ausgebildet ist, eine erste Erfassungskonfiguration umfassend eine Sendeantenne der Anzahl der Sendeantennen und eine Empfangsantenne der Anzahl der Empfangsantennen auszuwählen, und eine zweite Erfassungskonfiguration umfassend eine Sendeantenne der Anzahl der Sendeantennen und eine Empfangsantenne der Anzahl von Empfangsantennen auszuwählen, einem Verlustdetektor, welcher ausgebildet ist, bei Auswahl der ersten Erfassungskonfiguration zum Aussenden eines Sendesignals eine erste Verlustgröße auf der Basis des Sendesignals und eines Empfangssignals zu erfassen, und bei Auswahl der zweiten Erfassungskonfiguration zum Aussenden eines Sendesignals eine zweite Verlustgröße auf der Basis des Sendesignals und eines Empfangssignals zu erfassen, wobei der Prozessor ausgebildet ist, die Erfassungskonfiguration mit der geringeren Verlustgröße zum Erfassen des Blutbildparameters auszuwählen.

Bevorzugt wird bei der Auswahl der jeweiligen Erfassungskonfiguration das Blutgefäß erregt, wobei die Sendesignale beispielsweise in Richtung des Blutgefäßes ausgesendet werden. Auf der Basis der Empfangssignale, welche empfangene Versionen der Sendesignale sind, sowie auf der Basis der Sendesignale kann beispielsweise dasjenige Antennenpaar umfassend eine Sendeantenne und eine Empfangsantenne als diejenige Erfassungskonfiguration ausgewählt werden, die mit den geringsten Einkoppelverlusten verbunden ist. Die Einkoppelverluste können beispielsweise auf der Basis eines Vergleichs der vorgenannten Verlustgrößen, beispielsweise Absorptionslinien oder Dämpfungen, erfasst werden.

Gemäß einem Aspekt der Erfindung wird ein Verfahren zum Erfassen eines Parameters eines Blutbestandteils von Blut in einem Blutgefäß vorgeschlagen. Dabei werden ein erstes Sendesignal mit einer ersten Frequenz und ein zweites Sendesignal mit einer zweiten Frequenz in das Blutgefäß eingekoppelt. Weiter werden ein erstes Empfangssignal bei der ersten Frequenz und ein zweites Empfangssignal bei der zweiten Frequenz empfangen. Eine erste Verlustgröße wird auf der Basis des ersten Sendesignals und des ersten Empfangssignals ermittelt. Entsprechend wird eine zweite Verlustgröße auf der Basis des zweiten Sendesignals und des zweiten Empfangssignals ermittelt. Ferner wird eine Relaxationszeitkonstanten ($\tau$) des Blutbestandteils in Abhängigkeit von der Frequenz mit der größeren Verlustgröße ermittelt.

Gemäß einer bevorzugten Ausgestaltung hat das Verfahren folgende Schritte:

Einkoppeln zumindest eines Hochfrequenz-Signals mit einer Vielzahl von Frequenzen in das Blutgefäß,
Bestimmen der Frequenz, bei welcher der Imaginärteil der komplexen Dielektrizitätszahl ($\varepsilon''$) maximal ist,
Ermitteln der Relaxationszeitkonstanten ($\tau$) in Abhängigkeit der bestimmten Frequenz, und
Ermitteln des Blutbildparameters in Abhängigkeit der bestimmten Relaxationszeitkonstanten ($\tau$).

Weiters wird ein Computerprogrammprodukt vorgeschlagen, welches auf einer programmgesteuerten Einrichtung die Durchführung zumindest eines Teils des wie oben erläuterten Verfahrens zum Erfassen eines Parameters eines Blutbestandteils von Blut in einem Blutgefäß veranlasst. Der zumindest eine Teil, der als Computerprogrammprodukt ausgeführt ist, umfasst insbesondere den Schritt des Ermittelns der Relaxationszeitkonstanten ($\tau$).

Ein Computerprogrammprodukt wie ein Computerprogramm-Mittel kann beispielsweise als Speichermedium, wie Speicherkarte, USB-Stick, Floppy, CD-ROM, DVD oder auch in Form einer herunterladbaren Datei von einem Server in einem Netzwerk bereitgestellt oder geliefert werden. Dies kann zum Beispiel in einem drahtlosen Kommunikationsnetzwerk durch die Übertragung einer entsprechenden Datei mit dem Computerprogramm-Produkt oder dem Computerprogramm-Mittel erfolgen.

Weitere Ausführungsbeispiele werden Bezug nehmend auf die beiliegenden Zeichnungen erläutert. Es zeigen:

Fig. 1          ein schematisches Blockdiagramm einer Erfassungsvorrichtung;

Fig. 2          ein Diagramm zur Illustrierung der reellen Dielektrizitätszahl $\varepsilon'$ und der komplexen Dielektrizitätszahl $\varepsilon''$ in Abhängigkeit der Frequenz;

Fig. 3          ein Diagramm zur Illustrierung eines Zusammenhangs zwischen der Relaxationszeitkonstanten ($\tau$) und der Glucosekonzentration im Blut;

Fig. 4          ein schematisches Blockdiagramm einer Erfassungsvorrichtung mit einer Kommunikationsvorrichtung;

Fig. 5          ein schematisches Ablaufdiagramm eines Verfahrens zum Erfassen eines Blutbildparameters von Blut in einem Blutgefäß;

Fig. 6          ein schematisches Blockdiagramm eines Armbandes;

Fig. 7          ein schematisches Blockdiagramm eines Ausschnittes eines Ausführungsbeispiels eines Armbandes;

Fig. 8          ein schematisches Blockdiagramm eines Ausschnittes eines Armbandes;

Fig. 9          ein schematisches Blockdiagramm einer Anordnung der Elektroden der Erfassungsvorrichtung;

Fig. 10         ein schematisches Ablaufdiagramm eines Verfahrens zum Betreiben eines Armbandes;

Fig. 11         ein Blockdiagramm einer Erfassungsvorrichtung;

Fig. 12         ein Modell eines Querschnitts eines menschlichen Unterarms;

Fig. 13A-13D    Antennen;

Fig. 14         eine elektrische Dipolantenne;

Fig. 14B        eine Erregeranordnung;

Fig. 15A, 15B   Erregeranordnungen;

Fig. 16A        eine Schleifenantenne;

Fig. 16B      eine Erregeranordnung;

Fig. 17      eine Erregeranordnung;

Fig. 18      eine Erregeranordnung;

Fig. 19      eine Erregeranordnung;

Fig. 20      eine Erregeranordnung;

Fig. 21      ein Blockdiagramm einer Erfassungsvorrichtung;

Fig. 22      eine Frequenzverschiebung eines Absorptionsmaximums;

Fig. 23      ein Transmissionsverhalten;

Fig. 24      Frequenzverschiebungen;

Fig. 25      ein Diagramm eines Verfahrens zum Erfassen eines Blutbildparameters; und

Fig. 26      ein Blockdiagramm einer Erfassungsvorrichtung

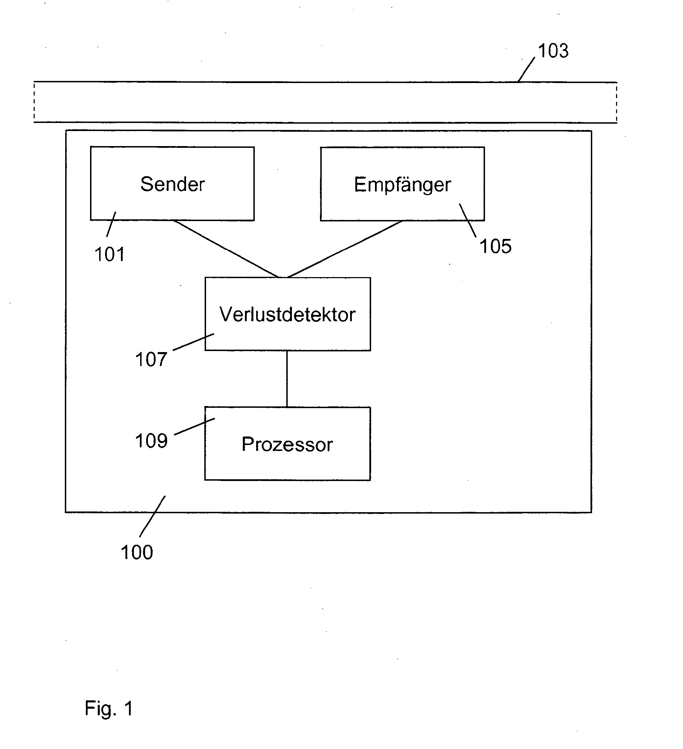

Fig. 1 zeigt ein Blockdiagramm einer Erfassungsvorrichtung 100 zum Erfassen eines Blutbildparameters, wie beispielsweise einer Konzentration von Blutzucker oder Glucose. Die Erfassungsvorrichtung 100 umfasst einen Sender 101, welcher ausgebildet ist, in das in Fig. 1 schematisch dargestellte Blutgefäß 103 ein erstes Sendesignal mit einer ersten Frequenz und ein zweites Sendesignal mit einer zweiten Frequenz einzukoppeln. Das erste Sendesignal und das zweite Sendesignal können beispielsweise gemeinsam ein Breitbandsignal ergeben. Der Sender 101 kann weiter dazu ausgebildet sein, das erste Sendesignal und das zweite Sendesignal sequenziell hintereinander in das Blutgefäß 103 einzukoppeln. Hierzu kann der Sender 101 eine oder mehrere Sendeantennen aufweisen, welche beispielsweise als Dipolantennen ausgebildet sind.

Die Erfassungsvorrichtung 100 umfasst ferner einen Empfänger 105, welcher ausgebildet ist, ein erstes Empfangssignal bei der ersten Frequenz und ein zweites Empfangssignal bei der zweiten Frequenz zu empfangen. Hierzu kann der Empfänger 105 eine oder mehrere Empfangsantennen aufweisen.

Des Weiteren hat die Erfassungsvorrichtung 100 einen Verlustdetektor 107, welcher beispielsweise mit dem Sender 101 und dem Empfänger 105 gekoppelt ist und dazu vorgesehen ist, eine erste Verlustgröße auf der Basis des ersten Sendesignals und des ersten Empfangssignals sowie eine zweite Verlustgröße auf der Basis des zweiten Sendesignals und des zweiten Empfangssignals zu bestimmen.

Die Erfassungsvorrichtung 100 weist ferner einen Prozessor 109 auf, welcher mit dem Verlustdetektor 107 gekoppelt ist und dazu vorgesehen ist, eine Relaxationszeitkonstante $\tau$ des Blutbildparameters in Abhängigkeit von der Frequenz mit der größeren Verlustgröße zu bestimmen.

Beispielsweise wird der Prozessor 109 die Relaxationszeitkonstante des Blutbildparameters in Abhängigkeit von der ersten Frequenz bestimmen, wenn die erste Verlustgröße größer als die zweite Verlustgröße ist. Entsprechend wird der Prozessor 109 die Relaxationszeitkonstante ($\tau$) des Blutbildparameters in Abhängigkeit von der zweiten Frequenz bestimmen, wenn die zweite Verlustgröße größer als die erste Verlustgröße ist.

Die in Fig. 1 dargestellte Erfassungsvorrichtung 100 nutzt die Erkenntnis aus, dass ein Blutgefäß, wie beispielsweise eine Vene, eine Hautschicht bzw. eine Vene umgebendes Fettgewebe als ein dielektrischer Wellenleiter aufgefasst werden kann. Der Aufbau eines menschlichen Unterarms ist in Netter, F.N., Atlas der Anatomie, Thieme-Verlag, 2006, beschrieben. Demnach besteht ein menschlicher Unterarm im Querschnitt aus zwei Knochen, welche von einem Muskelgewebe umgeben sind. Um das Muskelgewebe herum sind oberflächliche Venen verteilt. Die Knochen, das Muskelgewebe sowie die Venen sind durch ein Fettgewebe ummantelt, welches durch obere Hautschichten bedeckt ist. Die oberflächlichen Venen sind relativ nahe an den oberen Hautschichten angeordnet und von diesen durch das Fettgewebe separiert.

Werden beispielsweise der in Fig. 1 dargestellte Sender 101 und der Empfänger 105 auf die obere Hautschicht aufgelegt, so kann der Sender 101 dazu eingesetzt werden, in den durch eine Vene, ein Fettgewebe sowie eine Hautschicht gebildeten dielektrischen Wellenleiter eine transversal-elektrische (TE) oder eine transversal-magnetische (TM) Welle einzukoppeln. Dabei kann die Hautschicht sowie das Fettgewebe als ein Filmwellenleiter verstanden werden.

Wie oben bereits ausgeführt, ist der Verlustdetektor 107 dazu eingerichtet, eine erste Verlustgröße auf der Basis des ersten Sendesignals und des ersten Empfangssignals zu ermitteln und eine zweite Verlustgröße auf der Basis des zweiten Sendesignals und des zweiten Empfangssignals zu ermitteln. Bei Verwendung weiterer Sendesignal- und Empfangssignalpaare wird der Verlustdetektor 107 entsprechend weitere Verlustgrößen ermitteln.

Insbesondere ist der Verlustdetektor 107 dazu ausgebildet, die Verlustgrößen durch eine Zweitormessung zu bestimmen. Der Verlustdetektor 107 umfasst beispielsweise einen Netzwerkanalysator oder einen Leistungsdetektor.

Des Weiteren ist der Verlustdetektor 107 dazu eingerichtet, zur Bestimmung der Verlustgrößen jeweils einen Vorwärtstransmissionsfaktor $S_{21}$ und einen Eingangsreflexionsfaktor $S_{11}$ zu bestimmen.

Dabei wird der Verlustdetektor die jeweilige Verlustgröße $P_{Verlust}$ mittels folgender Formel berechnen:

$$P_{Verlust} = 1 - |S_{11}|^2 - |S_{21}|^2.$$

Insbesondere ist der Verlustdetektor 107 dazu eingerichtet, zur Bestimmung der jeweiligen Verlustgröße die komplexe Dielektrizitätszahl $\varepsilon''$ zu ermitteln.

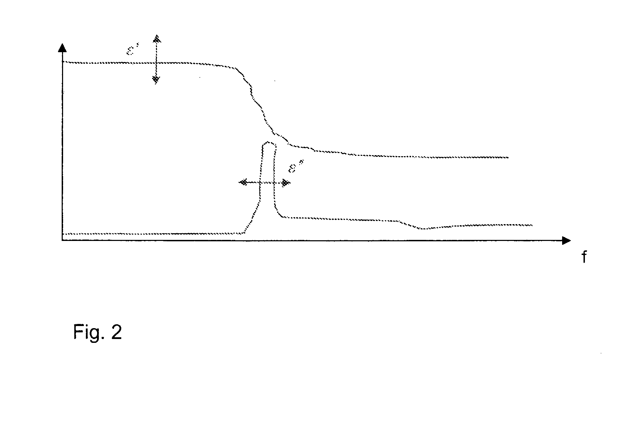

Hierzu zeigt die Fig. 2 ein Diagramm zur Illustrierung der reellen Dielektrizitätszahl $\varepsilon'$ und der komplexen Dielektrizitätszahl $\varepsilon''$ in Abhängigkeit der Frequenz f.

Dabei illustriert die Fig. 2, dass in dem Frequenzbereich, wo der Realteil $\varepsilon'$ von dem höheren Niveau auf das niedrigere Niveau übergeht, die durch die komplexe Dielektrizitätszahl $\varepsilon''$ repräsentierten Verluste steigen. Dieser Anstieg der Verluste wird in der Spektroskopie auch als Absorptionslinien bezeichnet. Der Effekt, der hierbei ausgenützt werden kann, ist der, dass sich die Frequenz, bei der die Überhöhung der Verluste - siehe lokales Maximum von $\varepsilon''$ - mit der Konzentration des Zuckergehaltes verschiebt.

Beispielsweise der menschliche Körper besteht zu 80% aus Wasser. Wasser besitzt Absorptionslinien, z.B. bei 19 GHz und 50 GHz. Deren Verstimmung kann bestimmt werden und auf den Zuckergehalt abgebildet werden. Die Verstimmung der Resonanzfrequenz bei $\varepsilon''$ ist - wie Fig. 2 illustriert - leichter detektierbar als die Plateauveränderung von $\varepsilon'$. Insbesondere verschieben Variationen in der Ankopplung die Frequenz des Maximums von $\varepsilon''$ vorteilhafterweise nicht. Damit ist eine Bestimmung der Zuckerkonzentration aus der Beobachtung von $\varepsilon''$ deutlich weniger fehleranfällig als die Beobachtung von $\varepsilon'$ bzw. dessen Niveauänderungen.

Weil bei einer Vielzahl von Stoffen solche Kurven wie die der Fig. 2 überlagert sind, ist eine Separation der Stoffe durch eine Beobachtung der imaginären Dielektrizitätszahl $\varepsilon''$ einfacher durchführbar, da jedem Stoff ein bestimmtes Absorptionsmaximum zugeordnet werden kann. Bei der reellen Dielektriziätszahl $\varepsilon'$ kann jedoch nur die Summe aller reellen Dielektrizitätszahlen $\varepsilon'$ aller beteiligten Stoffe beobachtet werden.

Wie oben bereits ausgeführt, ist der Prozessor 109 dazu ausgebildet, die Relaxationskonstante $\tau$ des Blutbildparameters in Abhängigkeit von der Frequenz mit der größeren oder maximalen Verlustgröße zu bestimmen. Weiter ist der Prozessor 109 dazu ausgebildet, den Blutbildparameter, wie die Glucosekonzentration im Blut, in Abhängigkeit der bestimmten Relaxationszeitkonstanten zu ermitteln.

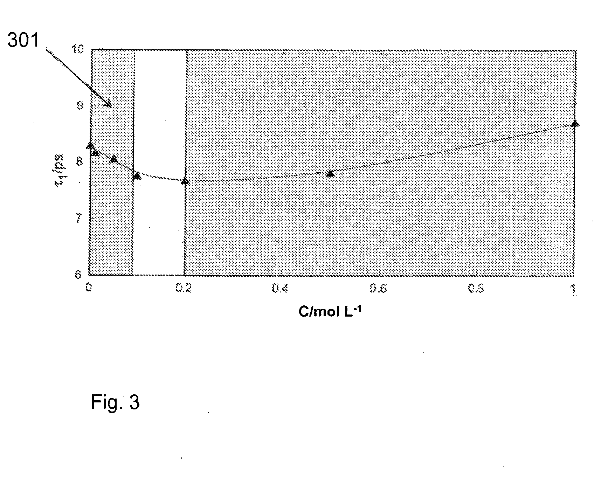

Dazu zeigt die Fig. 3 ein Diagramm zur Illustrierung eines Zusammenhangs zwischen der Relaxationszeitkonstanten ($\tau$) und der Glucosekonzentration C/mol L$^{-1}$ im Blut. Dabei zeigt der mit dem Bezugszeichen 301 referenzierte Bereich in der Fig. 3 einen kritischen Blutzuckerbereich.

Ferner ist der Prozessor 109 insbesondere dazu ausgebildet, die Relaxationszeitkonstante ($\tau$) auf der Basis der Formel $\tau = \dfrac{1}{2\pi f_A}$; zu berechnen, wobei $f_A$ die Frequenz bezeichnet, bei welcher die ermittelte Verlustgröße maximal ist.

Vorteilhafterweise ist dann der Prozessor 109 dazu ausgebildet, die Frequenz zu bestimmen, bei welcher der Imaginärteil der komplexen Dielektrizitätszahl $\varepsilon''$ maximal ist, und die Relaxationszeitkonstante ($\tau$) in Abhängigkeit der bestimmten Frequenz zu ermitteln ist. Diese bestimmte Frequenz nutzt der Prozessor 109 dann zur Bestimmung des Blutbildparameters, wie der Glucosekonzentration.

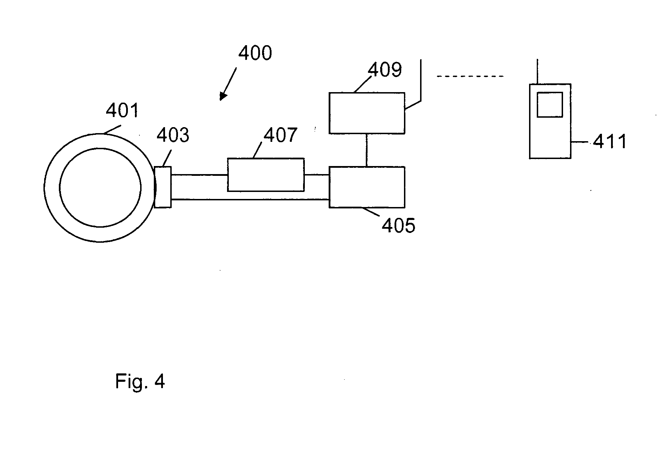

Die Fig. 4 zeigt ein schematisches Blockdiagramm einer Erfassungsvorrichtung 400. Die Erfassungsvorrichtung 400 hat ein Armband 401, einen an dem Armband 401 befestigten Sensor-Array 403, einen Mikroprozessor 405, einen Mikrowellenschaltkreis 407 zur Generierung der Sendesignale und eine Kommunikationsvorrichtung 409.

Der Sensor-Array 403 hat beispielsweise einen Mikrowellensensor, einen Temperatursensor und einen Feuchtigkeitssensor.

Der Mikroprozessor 405 ist beispielsweise wie der Prozessor 109 der Fig. 1 ausgebildet.

Die Kommunikationsvorrichtung 409 ist dazu eingerichtet, eine Kommunikationsverbindung für die Erfassungsvorrichtung 400 zu einer weiteren Kommunikationsvorrichtung 411 bereitzustellen. Die Kommunikationsvorrichtung 409 umfasst beispielsweise eine Bluetooth-Schnittstelle. Die weitere Kommunikationsvorrichtung 411 ist beispielsweise ein Mobilfunkgerät, ein Smartphone oder eine GPS-basierte Einrichtung.

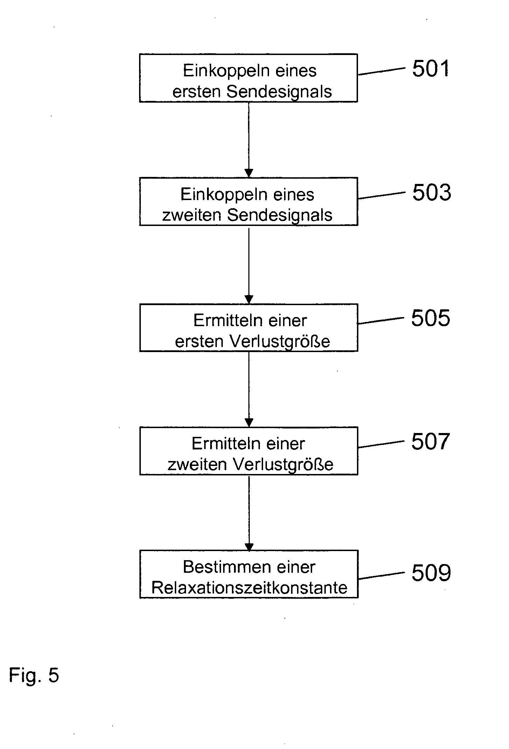

In Fig. 5 ist ein schematisches Ablaufdiagramm eines Ausführungsbeispiels eines Verfahrens zum Erfassen eines Blutbildparameters, wie beispielsweise einer Glucosekonzentration, von Blut in einem Blutgefäß dargestellt.

In Schritt 501 werden ein erstes Sendesignal mit einer ersten Frequenz und ein zweites Sendesignal mit einer zweiten Frequenz in das Blutgefäß eingekoppelt.

In Schritt 503 werden ein erstes Empfangssignal bei der ersten Frequenz und ein zweites Empfangssignal bei der zweiten Frequenz empfangen.

In Schritt 505 wird eine erste Verlustgröße auf der Basis des ersten Sendesignals und des ersten Empfangssignals ermittelt.

In Schritt 507 wird eine zweite Verlustgröße auf der Basis des zweiten Sendesignals und des zweiten Empfangssignals ermittelt.

In Schritt 509 wird eine Relaxationszeitkonstante des Blutbildparameters in Abhängigkeit von der Frequenz mit der größeren Verlustgröße bestimmt. Abhängig von der bestimmten Relaxationszeitkonstanten kann dann beispielsweise die Glucosekonzentration in dem Blut bestimmt werden.

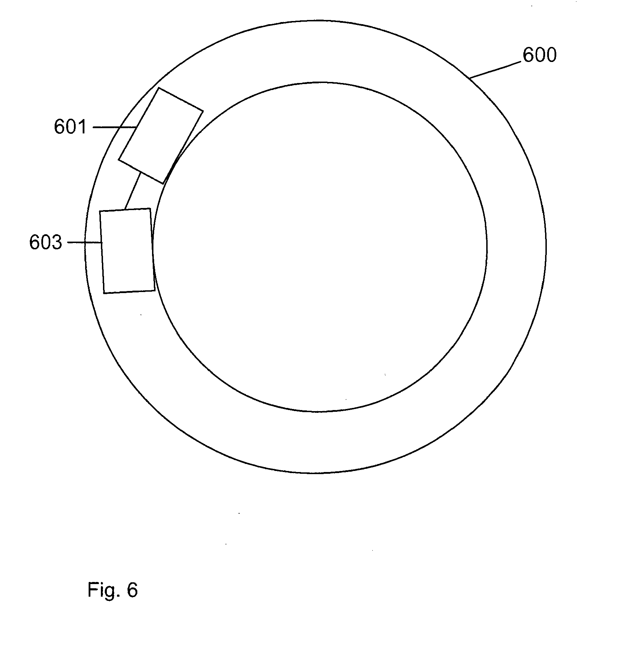

Die Fig. 6 zeigt ein Blockdiagramm eines Ausführungsbeispiels eines Armbandes 600 mit einer Erfassungsvorrichtung 601 und einer Einstellvorrichtung 603. Die Erfassungsvorrichtung 601 ist zum Erfassen eines Blutbildparameters von Blut in einem Blutgefäß des Armes eingerichtet. Ein Beispiel für den zu erfassenden Blutbildparameter ist die Glukosekonzentration in dem Blut.

Die Einstellvorrichtung 603 ist zum Einstellen eines vorgebbaren Anpressdruckes des Armbandes 600 auf den Arm eingerichtet. Durch Einstellen des vorgegebenen Anpressdruckes des Armbandes 600 kann die Einstellvorrichtung 603 reproduzierbare Erfassungen des Blutbildparameters durch die Erfassungsvorrichtung 601 sicherstellen. Dazu ist die Einstellvorrichtung 603 insbesondere dazu eingerichtet, den Anpressdruck des Armbandes 600 während des Erfassens des Blutbildparameters durch die Erfassungsvorrichtung 601 auf den vorgebbaren Anpressdruck einzustellen.

Das Armband 600 ist insbesondere als ein aufblasbares Armband 600 ausgebildet. Dabei hat die Einstellvorrichtung 603 insbesondere eine Luftpumpe, welche dazu ausgebildet ist, das Armband 600 zum Einstellen des vorgegebenen Anpressdruckes aufzublasen.

Im Detail umfasst die Erfassungsvorrichtung 601 insbesondere Elektroden, welche zur Einkopplung zumindest eines Hochfrequenzsignals in das Blutgefäß eingerichtet sind. Das Hochfrequenzsignal ist dazu ausgebildet, einen Parameter zur Erfassung des Blutbildparameters zu liefern. Ein Beispiel für einen solchen Parameter bildet die Relaxationszeitkonstante $\tau$ des Blutbildparameters. Dabei ist die Einstellvorrichtung 603 insbesondere dazu ausgebildet, den Anpressdruck der Elektroden auf den Arm auf den vorgegebenen Anpressdruck einzustellen.

Des Weiteren kann die Einstellvorrichtung 603 derart ausgebildet werden, dass sie die Anpresskräfte des Armbandes 600 während des Erfassens des Blutbildparameters durch die Erfassungsvorrichtung 601 gleichmäßig auf den Arm verteilt. Ferner ist die Einstellvorrichtung 603 vorzugsweise derart ausgebildet, dass sie ein gleichmäßiges Anliegen des Armbandes 600 während des Erfassens des Blutbildparameters durch die Erfassungsvorrichtung 601 sicherstellt.

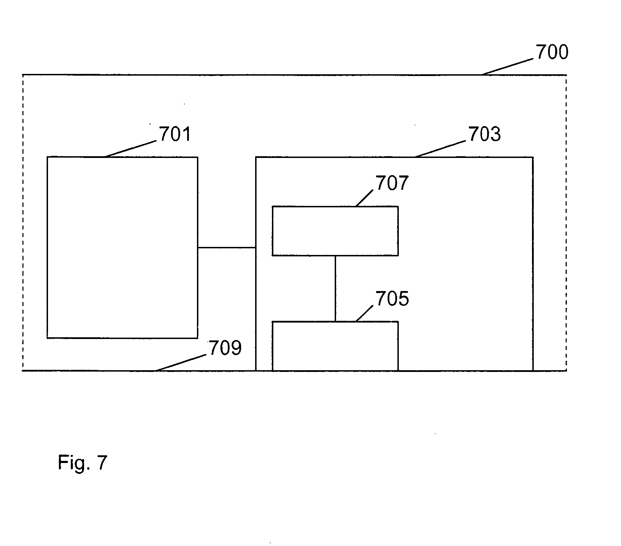

Die Fig. 7 zeigt ein Blockschaltbild eines Ausschnittes eines Ausführungsbeispiels eines Armbandes 700. Das Armband 700 hat eine Erfassungsvorrichtung 701 und eine Einstellvorrichtung 703. Die Erfassungsvorrichtung 701 und die Einstellvorrichtung 703 sind zumindest wie die Erfassungsvorrichtung 601 und die Einstellvorrichtung 603 der Fig.6 ausgebildet. Des Weiteren hat die Einstellvorrichtung 703 der Fig. 7 eine Sensoreinrichtung 705 und eine Steuereinrichtung 707. Die Sensoreinrichtung 705 ist dazu eingerichtet, einen aktuellen Anpressdruck des Armbandes 700 auf den Arm zu messen. In Abhängigkeit des gemessenen aktuellen Anpressdruckes stellt die Steuereinrichtung 707 den vorgegebenen Anpressdruck auf den Arm ein.

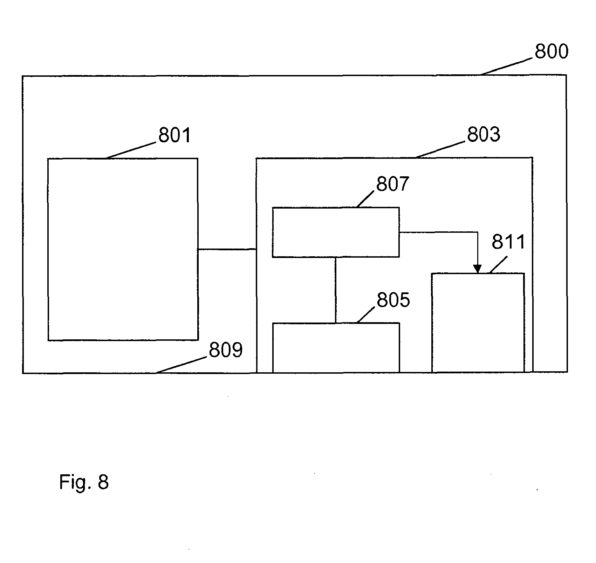

Die Fig. 8 zeigt ein Blockschaltbild eines Ausschnittes eines weiteren Ausführungsbeispiels eines Armbandes 800. Das Armband 800 hat eine Erfassungsvorrichtung 801 und eine Einstellvorrichtung 803. Die Einstellvorrichtung 803 hat eine Sensoreinrichtung 805, eine Steuereinrichtung 807 und eine Luftpumpe 811. Die Sensoreinrichtung 805 misst einen aktuellen Anpressdruck des Armbandes 800 auf den Arm. Die Steuereinrichtung 807 stellt ein Steuersignal in Abhängigkeit des gemessenen aktuellen Anpressdruckes bereit. Mittels des bereitgestellten Steuersignals wird die Luftpumpe 811 zum Aufblasen des Armbandes 800 gesteuert.

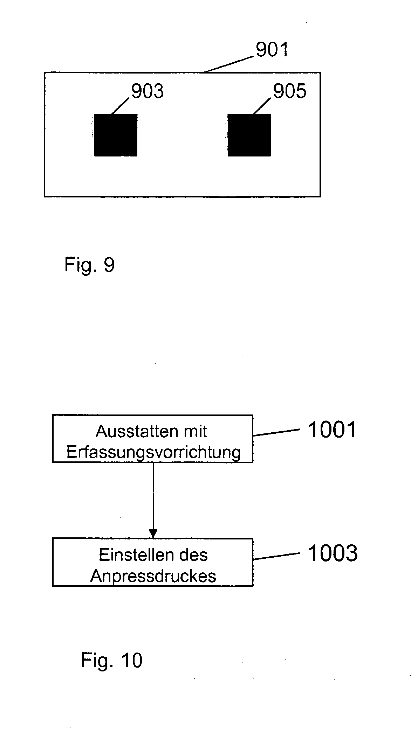

In Fig. 9 ist ein schematisches Blockdiagramm einer Anordnung 900 der Elektroden 903, 905 der Erfassungsvorrichtung zum Erfassen eines Blutbildparameters von Blut in einem Blutgefäß des Armes dargestellt

Ohne Einschränkung der Allgemeinheit zeigt die Anordnung 900 nur zwei Elektroden 903 und 905. Die Anordnung 900 ist insbesondere Teil der Erfassungsvorrichtung und beispielsweise als eine Platte mit beispielhaften Abmessungen von 5cm auf 2cm ausgestaltet. Die Elektroden 903, 905 haben beispielsweise eine Grundfläche von 5mm auf 5mm. Der Abstand der Elektroden 903, 905 beträgt beispielsweise 1 bis 2cm. Damit wird zum einen eine genügend starke Transmission erzielt und zum anderen wird eine genügend große Eindringtiefe in den Körper sichergestellt.

Die Fig. 10 zeigt ein schematisches Ablaufdiagramm eines Verfahrens zum Betreiben eines Armbandes mit einer Erfassungsvorrichtung.

In Schritt 1001 wird das Armband mit einer Erfassungsvorrichtung zum Erfassen eines Blutbildparameters von Blut in einem Blutgefäß des Armes ausgestattet. Die Erfassungsvorrichtung ist beispielsweise gemäß einem der Ausführungsbeispiele der Figuren 6, 7 oder 8 ausgebildet.

In Schritt 1003 wird ein vorgegebener Anpressdruck des Armbandes auf den Arm eingestellt. Somit wird ein reproduzierbares Erfassen des Blutbildparameters durch die Erfassungsvorrichtung sichergestellt.

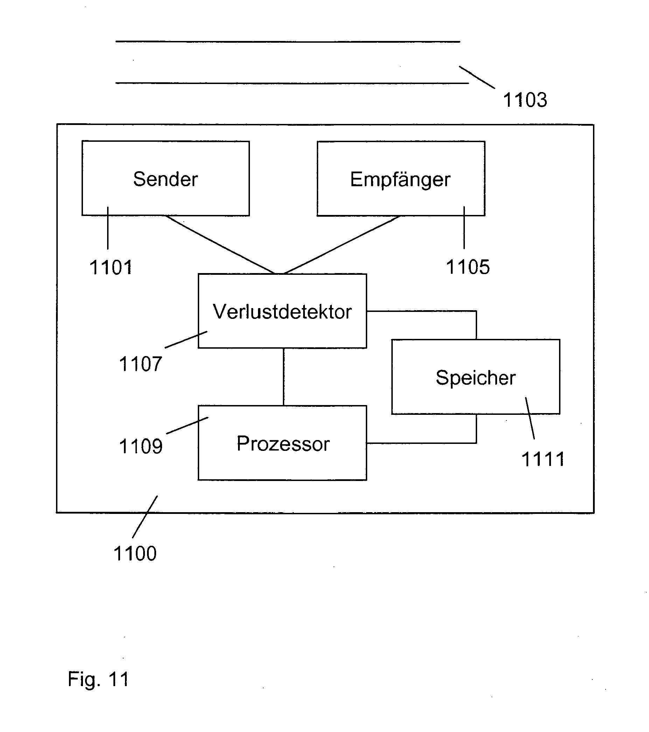

Fig. 11 zeigt ein Blockdiagramm einer Erfassungsvorrichtung 1100 zum Erfassen eines Blutbildparameters wie beispielsweise einer Konzentration von Blutzucker. Die Erfassungsvorrichtung 1100 umfasst einen Sender 1101, welcher ausgebildet ist, in das in Fig. 11 schematisch dargestellte Blutgefäß 1103 ein erstes Sendesignal mit einer ersten Frequenz und ein zweites Sendesignal mit einer zweiten Frequenz einzukoppeln. Das erste Sendesignal und das zweite Sendesignal können beispielsweise gemeinsam ein Breitbandsignal ergeben. Der Sender 1101 kann ausgebildet sein, das erste Sendesignal und das zweite Sendesignal hintereinander, beispielsweise durch einen Frequenz-Sweep, auszusenden. Hierzu kann der Sender 1101 eine oder mehrere Sendeantennen, welche beispielsweise als Dipolantennen oder Rahmenantennen oder Patch-Antennen ausgebildet sein können, aufweisen.

Die Erfassungsvorrichtung 1100 umfasst ferner einen Empfänger 1105, welcher ausgebildet ist, ein erstes Empfangssignal bei der ersten Frequenz und ein zweites Empfangssignal bei der zweiten Frequenz zu empfangen. Hierzu kann der Empfänger 1105 eine oder mehrere Empfangsantennen aufweisen.

Die Erfassungsvorrichtung 1100 umfasst ferner einen Verlustdetektor 1107, welcher beispielsweise mit dem Sender 1101 und dem Empfänger 1105 gekoppelt und vorgesehen ist, eine erste Verlustgröße auf der Basis des ersten Sendesignals und des ersten Empfangssignals sowie eine zweite Verlustgröße auf der Basis des zweiten Sendesignals und des zweiten Empfangssignals zu bestimmen.

Die Erfassungsvorrichtung umfasst ferner einen Prozessor 1109, welcher mit dem Verlustdetektor 1107 gekoppelt und vorgesehen ist, eine erste Frequenzverschiebung der ersten Verlustgröße relativ zu einer ersten Referenzverlustgröße und eine zweite Frequenzverschiebung der zweiten Verlustgröße relativ zu einer zweiten Referenzverlustgröße zu bestimmen. Der Prozessor 1109 kann ferner ausgebildet sein, den Blutbildparameter auf der Basis der beiden Frequenzverschiebungen zu bestimmen.

Die Erfassungsvorrichtung 1100 kann ferner einen Speicher 1111 aufweisen, auf welchen beispielsweise der Prozessor 1109 und optional der Verlustdetektor 1107 zugreifen können. In dem Speicher 1111 sind beispielsweise die erste und die zweite Referenzverlustgröße oder eine Mehrzahl von Referenzverlustgrößen abgelegt. Bei den Referenzverlustgrößen kann es sich beispielsweise um Absorptionen oder Absorptionslinien einer Wasserlösung mit einem Blutbestandteil, beispielsweise Blutzucker, handeln. Die auf der Basis der Frequenzverschiebungen erfassten Verlustgrößen können frequenzverschobene Absorptionen oder Absorptionslinien sein, so dass auf der Basis der Frequenzverschiebungen der Blutbildparameter, wie beispielsweise eine Konzentration von Blutzucker, ermittelt werden kann.

Die in Fig. 11 dargestellte Erfassungsvorrichtung 1100 nutzt die Erkenntnis aus, dass ein Blutgefäß, eine Hautschicht sowie ein das Blutgefäß umgebendes Fettgewebe von beispielsweise einem menschlichen Unterarms als ein dielektrisches Wellenleitersystem aufgefasst werden können. Werden beispielsweise der in Fig. 11 dargestellte Sender 1101 und der Empfänger 1105 auf die obere Hautschicht aufgelegt, so kann der Sender 1101 dazu eingesetzt werden, in das durch ein Blutgefäß, ein Fettgewebe sowie eine Hautschicht gebildete dielektrische Wellenleitersystem beispielsweise eine transversal-elektrische (TE) Welle oder eine transversal-magnetische (TM) Welle einzukoppeln. Dabei kann die Hautschicht sowie das Fettgewebe als ein Filmwellenleiter verstanden werden. Wird nun beispielsweise ein Mikrowellenmesskopf, wie er für die Bestimmung einer komplexen Dielektrizitätszahl von Materialien benutzt werden kann, verwendet, so kann dadurch das Stoffgemisch bestehend aus Haut, Fettgewebe und Venen charakterisiert werden.

Um einen Blutbildparameter zu erfassen, ist es vorteilhaft, im Wesentlichen nur das venöse Blut zu erfassen. Hierzu kann der Sender 1101 ausgebildet sein, das Sendesignal in der Gestalt einer elektromagnetischen Welle direkt in das Blutgefäß einzukoppeln. Der Sender 1101 sowie der Empfänger 1105 können jeweils eine Mehrzahl von Antennen aufweisen, so dass zur Einkopplung der elektromagnetischen Welle in das Blutgefäß und zur Auskopplung einer elektromagnetischen Welle aus dem Blutgefäß jeweils diejenige Sendeantenne und diejenige Empfangsantenne ausgewählt werden können, welche mit den geringsten Koppelverlusten verbunden sind.

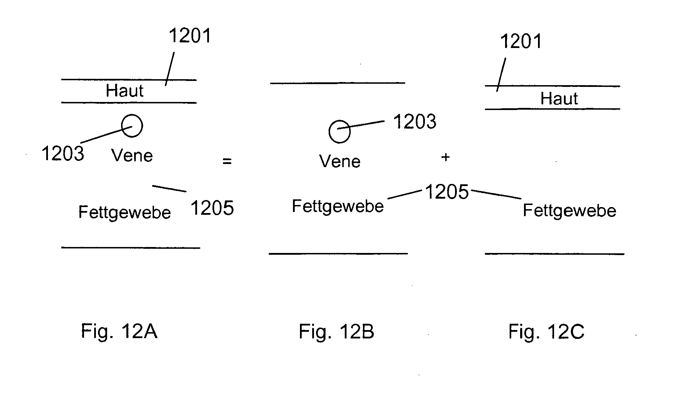

In den Figuren 12A bis 12C ist ein vereinfachtes Modell eines Querschnitts eines menschlichen Unterarms, z.B. eines Handgelenks, wie es beispielsweise für Feldsimulationen bzw. zur Modellierung eines dielektrischen Wellenleitersystems benutzt werden kann. Wie in Fig. 12A dargestellt, umfasst das Modell eine Hautschicht 1201, ein Blutgefäß 1203 sowie ein das Blutgefäß 1203 umgebendes Fettgewebe 1205. Das in Fig. 12A dargestellte Modell bildet ein dielektrisches Wellenleitersystem umfassend den in Fig. 12B dargestellten dielektrischen Wellenleiter sowie den in Fig. 12C dargestellten elektrischen Filmwellenleiter.

Der in Fig. 12B dargestellte dielektrische Wellenleiter umfasst das Blutgefäß 1203 sowie das dieses umgebende Fettgewebe 1205. Der dielektrische Filmwellenleiter aus Fig. 12C umfasst hingegen die Hautschicht 1201 sowie das Fettgewebe 1205. Für die Hautschicht 1201, das Fettgewebe 1205 sowie für das Blutgefäß 1203 kann jeweils ein unterschiedliches dispersives, d.h. frequenzabhängiges, Verhalten der jeweiligen komplexen Dielektrizitätszahl angesetzt werden. Das oben liegende Blutgefäß 1203 wird dabei als ein dielektrischer Wellenleiter interpretiert, in dem je nach Frequenz verschiedene Moden bzw. Wellentypen, beispielsweise eine TE-Welle, eine TM-Welle, eine TEM-Welle oder eine HE-Welle, ausbreitungsfähig sein können. Zu dem Wellenleitermechanismus in dem dielektrischen Wellenleiter kommt ein weiterer Wellenleitermechanismus in der Gestalt des in Fig. 12C dargestellten Filmwellenleiters hinzu, der durch die obere Hautschicht 1201 gebildet wird.

Eine Sendeantenne des Senders 1101 sowie eine Empfangsantenne des Empfängers 1105 können bevorzugt derart ausgestaltet sein, dass sie dezidiert Mikrowellenleistung in das Blutgefäß 1203 einkoppeln und diese beispielsweise nach einigen Zentimetern wieder auskoppeln. Das Blutgefäß 1203 dient dabei als eine Messstrecke und ist somit als ein verteiltes Element und nicht mehr als ein konzentriertes Element zu sehen. Bevorzugt wird die Messung der Verlustgrößen auf der Basis einer Zweitormessung durchgeführt. Dabei können insbesondere bei einer Ankopplung der Erfassungsvorrichtung an ein Handgelenk Primärmoden in dem dielektrischen Wellenleiter gemäß Fig. 12B angeregt werden, so dass eine Anregung von Filmwellenleitermoden in dem Filmwellenleiter gemäß Fig. 12C vermieden wird, so dass die Erfassung des Blutbildparameters genauer durchgeführt werden kann.

Um Primärmoden im dielektrischen Wellenleitersystem anzuregen, kann berücksichtigt werden, dass je nach gewählter Frequenz eines Sendesignals unterschiedliche Moden dominant sein können. Bevorzugt sind Modentypen, die eine Konzentration der Felder in der Vene 1203 aufweisen, solchen Moden vorzuziehen, bei denen die Felder in der Hautschicht 1201 konzentriert sind. Aufgrund der dielektrischen Eigenschaften des in Fig. 12B dargestellten dielektrischen Wellenleiters zeigt sich, dass für bestimmte Modentypen longitudinale Komponenten $E_{longitudinal}$, $H_{longitudinal}$ in Ausbreitungsrichtung, d.h. in Richtung eines Venenverlaufs, stärker als die transversalen Komponenten $E_{transversal}$, $H_{transversal}$, d.h. quer zum Venenverlauf, sind. Bevorzugt werden daher in dem zu erfassenden Frequenzbereich diejenigen Moden angeregt, welche eine maximale Einkopplung der Mikrowellenleistung in das Blutgefäß 1203 ermöglichen.

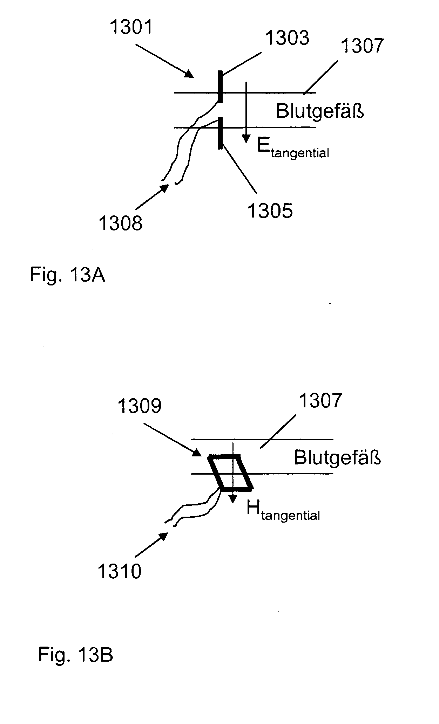

In den Figuren 13A bis 13D sind beispielhaft einige Antennen dargestellt, welche als Sendeantennen, d.h. Erreger, oder auch als Empfangsantennen eingesetzt werden können.

Die in Fig. 13A dargestellte Antenne 1301 ist als ein elektrischer Dipol mit einem ersten Antennenabschnitt 1303 und einem zweiten Antennenabschnitt 1305 ausgeführt Die Antennenabschnitte 1303 und 1305 sind voneinander beabstandet und beispielsweise quer zu einem Verlauf eines Blutgefäßes 1307 angeordnet. Eine Anregung der Antenne 1301 kann durch Zuleitungen 1308 erfolgen. Ein derart angeordneter elektrischer Dipol kann beispielsweise ein elektrisches Feld $E_{tangential}$ erzeugen, das quer zum Verlauf des Blutgefäßes bzw. zur Blutflussrichtung zeigt.

In Fig. 13B ist eine Antenne 1309 dargestellt, welche eine Rahmenantenne sein kann. Die Rahmenantenne kann beispielsweise eine Viereckform oder eine runde Form aufweisen. Bei der in Fig. 13B dargestellten Anordnung der Rahmenantenne 1309 bezüglich des Blutgefäßes 1307 wird beispielsweise ein magnetisches Feld $H_{tangential}$ angeregt, das quer zum Verlauf des Blutgefäßes 1307 bzw. quer zur Blutflussrichtung zeigt. Eine Anregung der Antenne 1309 kann durch Zuleitungen 1310 erfolgen.

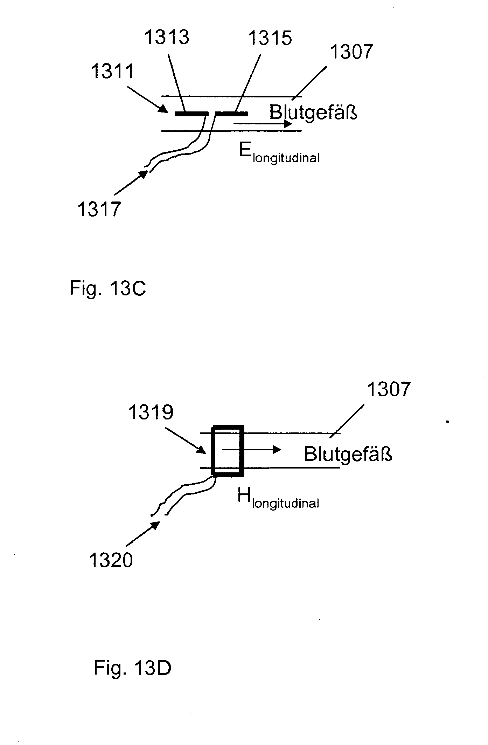

In Fig. 13C ist eine Antenne 1311 dargestellt, welche einen elektrischen Dipol mit einem ersten Antennenabschnitt 1313 und einem zweiten Antennenabschnitt 1315 bildet. Die Antennenabschnitte 1313 und 1315 sind voneinander beabstandet und werden mittels der in Fig. 13C dargestellten Zuleitungen 1317 angeregt. Der durch die Antenne 1311 gebildete elektrische Dipol ist nun derart bezüglich des Verlaufs des Blutgefäßes 1307 angeordnet, dass die Abschnitte 1313 und 1315 parallel zum Verlauf des Blutgefäßes 1307 angeordnet ist. Dadurch wird ein in Richtung des Verlaufs des Blutgefäßes weisendes elektrisches Feld mit der Feldkomponente $E_{longitudinal}$ angeregt.

Fig. 13D zeigt eine Rahmenantenne 1319, welche beispielsweise in der Gestalt eines viereckigen oder runden Rahmens, welcher eine Schleifenantenne bildet, beispielsweise als eine Patch-Antenne, gebildet sein kann. Die Rahmenantenne 1319 wird mittels Zuleitungen 1320 angeregt und ist wie es in Fig. 13D dargestellt ist, bezüglich des Verlaufs des Blutgefäßes 1307 bzw. bezüglich der Blutflussrichtung derart angeordnet, dass das magnetische Feld eine in Richtung des Verlaufs des Blutgefäßes 1307 weisende Komponente $H_{longitudinal}$ aufweist.

Der jeweils zu messende Frequenzbereich richtet sich beispielsweise danach, welche Spektrallinien, d.h. welche Absorptionslinien, zu erfassen sind. Es können beispielsweise die charakteristischen Absorptionslinien eines Stoffes oder auch ein Effekt beobachtet werden, den ein bestimmter Blutbestandteil auf die Absorptionslinien von Wasser bzw. von einer Wasserlösung mit einer Konzentration des Blutbestandteils aufweist.

Bei den in den Figuren 13A bis 13D dargestellten Antennen handelt es sich entweder um elektrische Dipole oder um magnetische Rahmenantennen. Darüber hinaus können auch Patch-Antennen eingesetzt werden. Elektrische Dipole erzeugen dominant ein elektrisches Feld in der Achse des elektrischen Dipols. Diese Achse kann entweder, wie es in Fig. 13A dargestellt ist, tangential zum Blutgefäß 1307 bzw. zur Blutflussrichtung, oder wie es in Fig. 13C dargestellt ist, in Richtung des Blutgefäßes 1307 bzw. in Blutflussrichtung ausgerichtet sein. Falls primär ein magnetisches Feld erzeugt werden soll, so kann eine Rahmenantenne als Erreger eingesetzt werden. Ist ein Flächenvektor auf der von dem die Rahmenantenne bildenden Rahmen aufgespannten Fläche quer zu dem Blutgefäß 1307 bzw. quer zur Blutflussrichtung ausgerichtet, so ist auch das magnetische Feld quer zu dem Blutgefäß 307 ausgerichtet, wie es in Fig. 13B dargestellt ist. Zeigt der Flächenvektor hingegen in Richtung des Blutgefäßes 1307, so ist auch das Magnetfeld in Richtung des Blutgefäßes 1307 ausgerichtet, wie es beispielsweise in Fig. 13B dargestellt ist. Aus der Wahl eines der in den Figuren 13A bis 13D dargestellten Erregers ergibt sich dann beispielsweise die dominant angeregte Mode bzw.

Wellentyp.

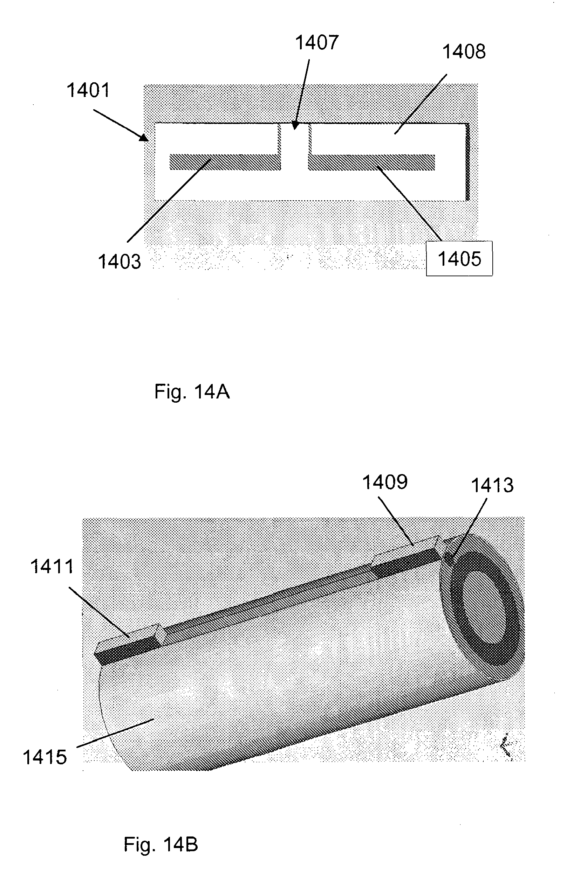

Fig. 14A zeigt eine elektrische Dipolantenne 1401, welche als eine Sendeantenne oder als eine Empfangsantenne eingesetzt werden kann. Die elektrische Dipolantenne 1401 umfasst Dipolantennenabschnitte 1403 und 1405, welche in einem Substrat 1408 angeordnet sind und mittels Zuleitungen 1407 angeregt werden können. Die Dipolantenne 1401 kann als Sendeantenne oder als Empfangsantenne eingesetzt werden.

Fig. 14B zeigt eine Erregeranordnung einer Sendeantenne 1409 eines Senders und einer Empfangsantenne 1411 eines Empfängers in Richtung eines Verlaufs eines Blutgefäßes 1413 unterhalb einer Hautschicht 1415. Die Sendeantenne 1409 und die Empfangsantenne 1411 sind beispielsweise elektrische Dipolantennen gemäß Fig. 14A. Bei der in Fig. 14B dargestellten Anordnung wird ein elektrisches Feld mit einer Feldkomponente in Richtung des Verlaufs des Blutgefäßes 1413, bzw. in Blutflussrichtung erzeugt.

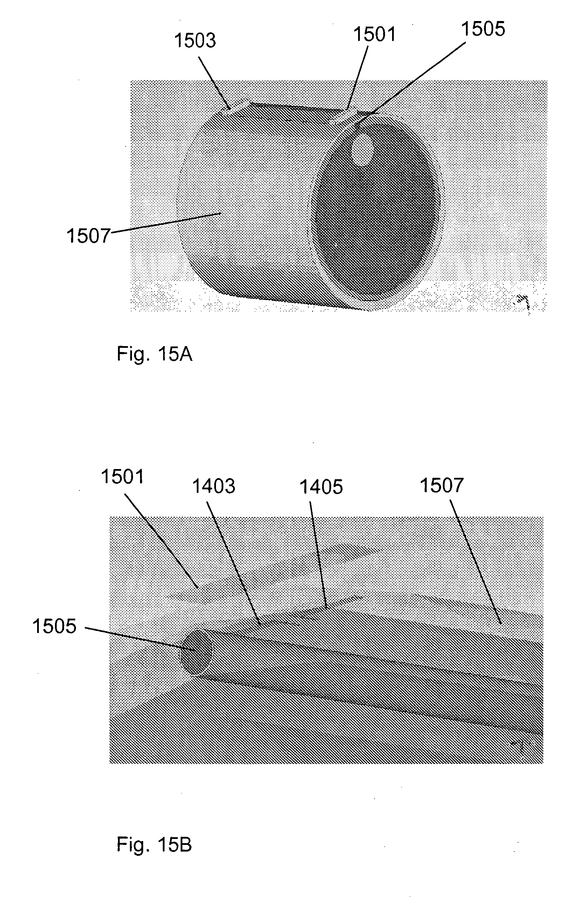

Fig. 15A zeigt eine Erregeranordnung umfassend eine Sendeantenne 1501 eines Senders und eine Empfangsantenne 1503 eines Empfängers quer zur Ausbreitungsrichtung eines Blutgefäßes 1505, d.h. quer zur Blutflussrichtung, das unter einer Hautschicht 1507 liegt. Die Sendeantenne 1501 und die Empfangsantenne 1503 können jeweils beispielsweise durch die in Fig. 14A dargestellte elektrische Dipolantenne gebildet sein. In Fig. 15B ist die Anordnung der Dipolantennenabschnitte 1403 und 1405 bezüglich der Blutflussrichtung genauer dargestellt.

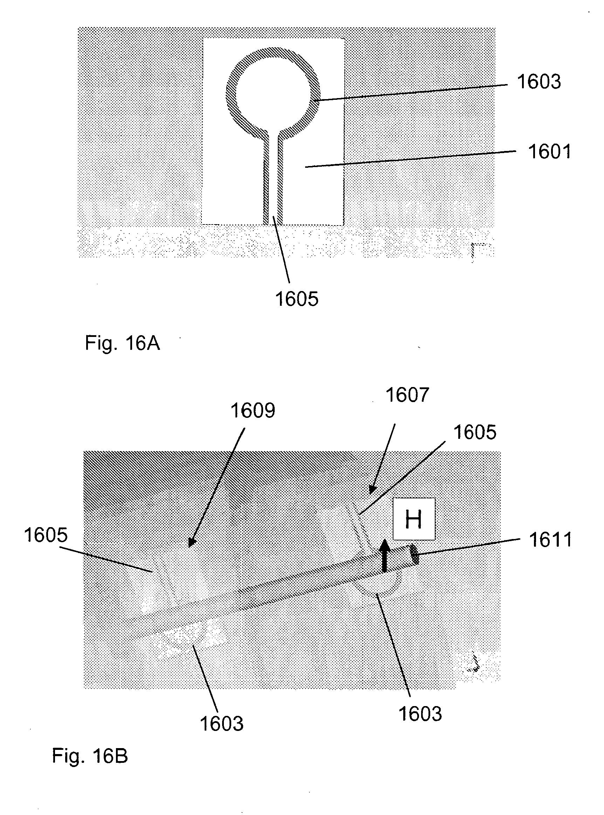

Fig. 16A zeigt eine Schleifenantenne 1601 mit einem kreisförmigen Rahmen 1603 und Zuleitungen 1605 zum Anregen des kreisförmigen Rahmens 1603. Die Schleifenantenne 1601 kann beispielsweise als eine Sendeantenne oder als eine Empfangsantenne eingesetzt werden. Der kreisförmigen Rahmen 1603 sowie die Zuleitungen 1605 können in einem Substrat angeordnet werden.

Fig. 16B zeigt eine Erregeranordnung mit einer Sendeantenne 1607 eines Senders und einer Empfangsantenne 1609 eines Empfängers, welche als Schleifenantennen gemäß Fig. 16A gebildet sein können. Die Schleifenantennen 1607, 1609 sind beispielsweise derart angeordnet, dass die kreisförmigen Rahmen 1603 oberhalb eines Blutgefäßes 1611 angeordnet sind, wobei die Zuleitungen 1605 quer zum Verlauf des Blutgefäßes 1611, d.h. quer zur Blutflussrichtung, zeigen. Dadurch wird senderseitig ein Magnetfeld H mit einer quer zum Verlauf des Blutgefäßes 1611 weisenden Komponente des Magnetfeldes erzeugt.

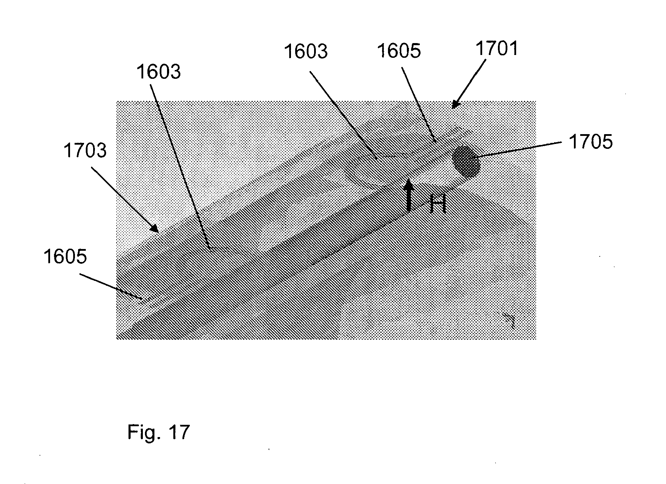

Fig. 17 zeigt eine Erregeranordnung einer Sendeantenne 1701 eines Senders und einer Empfangantenne 1703 eines Empfängers bezüglich eines Blutgefäßes 1705. Die Sendeantenne 1701 und die Empfangsantenne 1703 können beispielsweise Schleifenantennen mit der in Fig. 16A dargestellten Form sein. Sie sind beispielsweise derart angeordnet, dass die kreisrunden Rahmen 1603 jeweils oberhalb des Blutgefäßes 1705 angeordnet sind und dass die Zuleitungen 1605 voneinander weisend parallel zum Verlauf des Blutgefäßes 1705 verlaufen. Dadurch wird eine senkrecht zum Verlauf des Blutgefäßes 1705 weisende Feldkomponente H erzeugt, welche in Richtung einer Normalen der durch den kreisrunden Rahmen 1603 aufgespannten Fläche zeigt.

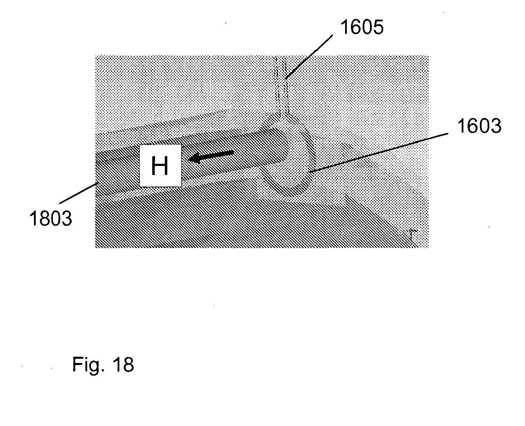

Fig. 18 zeigt eine Erregeranordnung mit einer Sendeantenne 1801 eines Senders, welche beispielsweise die in Fig. 16A dargestellte Form einer Schleifenantenne aufweist. Die Sendeantenne 1801 ist bezüglich eines Blutgefäßes 1803 beispielsweise derart angeordnet, dass eine Normale der durch den Rahmen 1603 aufgespannten Fläche in Richtung des Verlaufes des Blutgefäßes 1803 zeigt. Eine derartige Anordnung kann beispielsweise bei einem Knick des Blutgefäßes 1803 realisiert werden. Dadurch wird eine in Richtung des Verlaufes des Blutgefäßes 1803 weisende magnetische Feldkomponente H erzeugt.

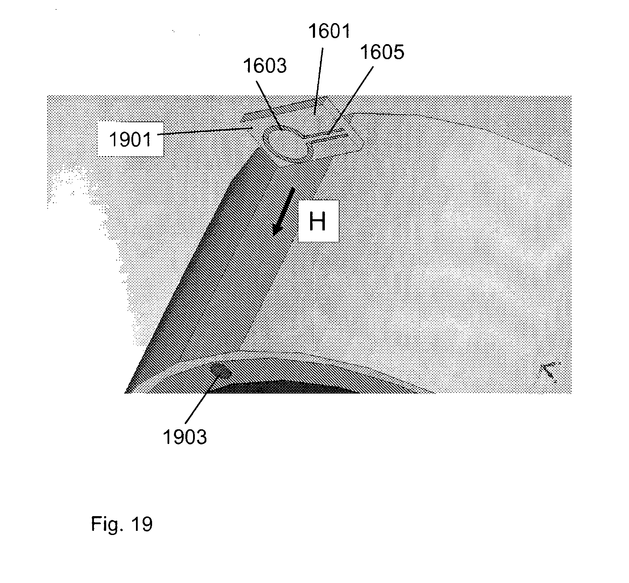

Fig. 19 zeigt eine Erregeranordnung mit einer Sendeantenne 1601, welche beispielsweise eine Schleifenantenne mit der in Fig. 16A dargestellten Form ist und in einem Substrat 1901, beispielsweise Kunststoffsubstrat, angeordnet sein kann. Die Sendeantenne 1601 ist oberhalb eines Blutgefäßes 1903 derart angeordnet, dass eine Normale der durch den kreisförmigen Rahmen 1603 aufgespannten Fläche in Richtung des Verlaufes des Blutgefäßes 1903 zeigt. Dadurch wird ein magnetisches Feld mit einer in Richtung des Verlaufes des Blutgefäßes 1903, d.h. in Blutflussrichtung, weisenden Feldkomponente H erzeugt.

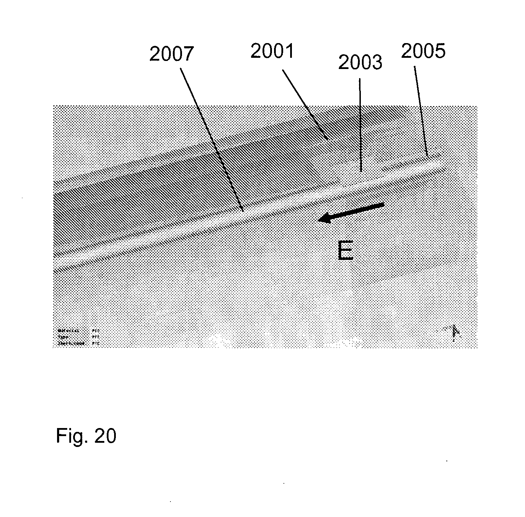

Fig. 20 zeigt eine Erregeranordnung mit einer Sendeantenne 2001, welche eine Patch-Antenne mit einer Patch-Antennenfläche 2003 und Zuleitungen 2005 sein kann. Die Patch-Antennenfläche 2003 ist beispielsweise oberhalb eines Blutgefäßes 2007 angeordnet, wodurch ein elektrisches Feld mit einer in Richtung eines Verlaufs des Blutgefäßes 2007, d.h. in Blutflussrichtung, weisenden elektrischen Feldkomponente E erzeugt wird.

Gemäß einer Ausführungsform ist der Verlustdetektor 1107 ausgebildet, beispielsweise eine skalare oder eine vektorielle Messung oder eine Leistungsmessung durchzuführen. Zur Bestimmung der Verlustgrößen kann eine einfache spektroskopische Messung durchgeführt werden, bei der der Betrag des Messparameters S21 erfasst wird.

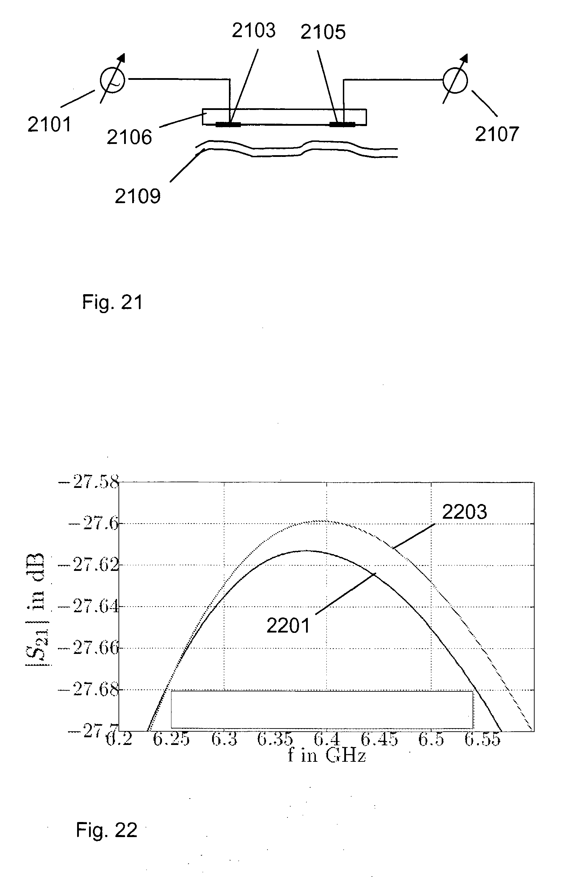

Die Messung von $|S_{21}|$ kann beispielsweise mittels der in Fig. 21 dargestellten Erfassungsvorrichtung durchgeführt werden. Die Erfassungsvorrichtung umfasst einen Sender mit einem Sendesignalgenerator 2101, welcher ein durchstimmbarer Oszillator sein kann. Ein Ausgang des Sendesignalgenerators 2101 ist mit einer Sendeantenne 2103 verbunden. Die Erfassungsvorrichtung umfasst ferner einen Empfänger mit einer Empfangsantenne 2105, deren Ausgang mit einem Verlustdetektor 2107 verbunden ist. Der Verlustdetektor kann beispielsweise einen Leistungsdetektor umfassen. Wie in Fig. 21 dargestellt ist, sind die Sendeantenne 2103 und die Empfangsantenne 2105 oberhalb eines Blutgefäßes 2109 angeordnet. Der Sender kann dem Sender 1101, der Empfänger kann dem Empfänger 1105 und der Verlustdetektor 2107 kann dem Verlustdetektor 1107 entsprechen.

Durch eine weitere Messung eines Betrags des Messparameters von S11 kann jedoch die Genauigkeit bei der Bestimmung der Verlustgrößen, d.h. der Verluste in dem Wellenleiter, noch weiter gesteigert werden. Die Verlustgrößen können beispielsweise auf der Basis der folgenden Formel bestimmt werden:

$$P_{Verlust} = 1 - \left| S_{11} \right|^2 - \left| S_{21} \right|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

Zum Erfassen des Blutbildparameters, beispielsweise einer Konzentration von Blutzucker, können beispielsweise Frequenzverschiebungen von Absorptionslinien einer Wasserlösung mit Zucker untersucht werden. Fig. 22 zeigt beispielsweise eine Frequenzverschiebung eines Absorptionsmaximums 2201 bei einer ersten Blutzuckerkonzentration im Vergleich zu einer Frequenzverschiebung eines Absorptionsmaximums 2203 bei einer zweiten Blutzuckerkonzentration, welche höher als die erste Blutzuckerkonzentration. Dabei wurde als Verlustgröße beispielhaft eine Transmission um 6 GHz erfasst.

Die Frequenzverschiebung des Absorptionsmaximums kann als ein Maß für einen Blutbildparameter, beispielsweise für einen Blutzuckerspiegel, aufgefasst werden. Durch eine Beobachtung von Frequenzverschiebungen bei mehreren Absorptionen einer Wasserlösung mit Zucker kann die Messzuverlässigkeit noch weiter erhöht werden.

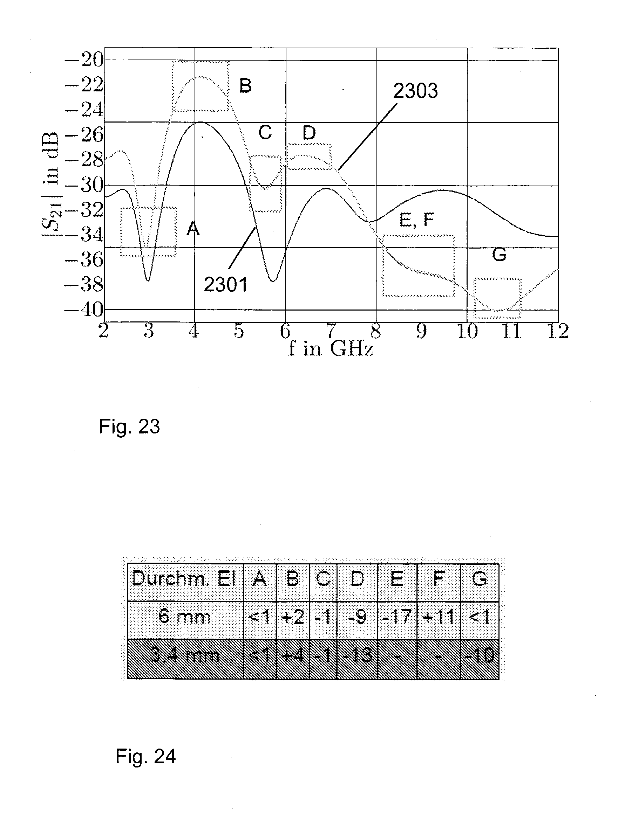

Fig. 23 zeigt beispielhaft ein breitbandiges Transmissionsverhalten von venösem Blut in einem Handgelenk. Dabei verdeutlichen die Verläufe 2301 und 2303 unterschiedliche Frequenzlagen von Absorptionslinien bei unterschiedlichen Blutzuckerkonzentrationen. Zur Erfassung des Blutbildparameters, wie beispielsweise der Konzentration des Blutzuckers, können beispielsweise gezielt Frequenzverschiebungen der Absorptionen A, B, C, D, E, F und G erfasst werden. So kann beispielsweise für jede Frequenz eines Absorptionsmaximums und/oder eines Absorptionsminimums eine Verschiebung in Richtung höherer oder niedriger Frequenzen je nach Blutzuckerspiegel beispielsweise in einem Frequenzbereich zwischen 2 GHz und 12 GHz beobachtet werden.

Fig. 24 zeigt beispielhaft Frequenzverschiebungen der in Fig. 23 dargestellten Absorptionen A, B, C, D, E, F und G für ein Blutgefäß mit 6 mm Durchmesser und für ein Blutgefäß mit 3,4 mm Durchmesser. Zu erkennen ist, dass die Absorptionen für eine Zuckerspiegelvariation Frequenzverschiebungen sowohl in positiver als auch in negativer Richtung aufweisen können. Die Erfassung von mehreren Absorptionen bzw. Absorptionslinien ermöglicht somit eine genauere Erfassung eines Blutbildparameters, beispielsweise des Blutzuckerspiegels.

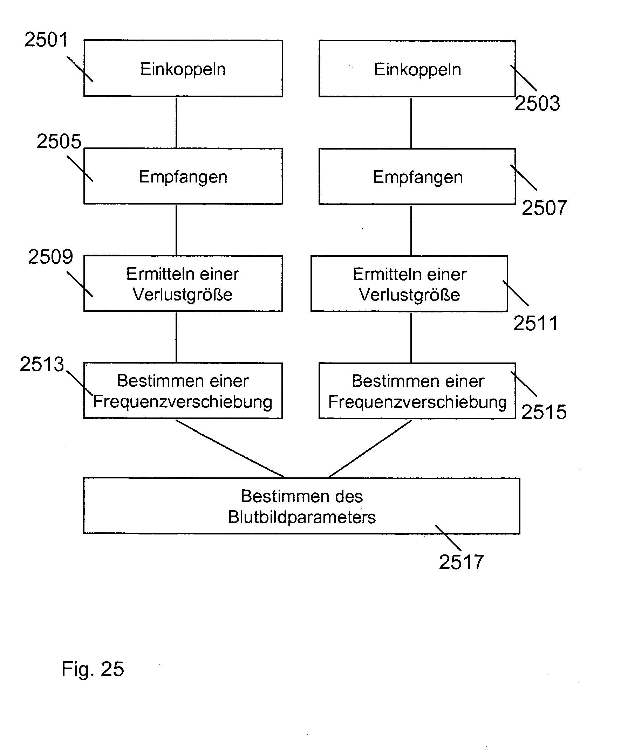

Fig. 25 zeigt ein Diagramm eines Verfahrens zum Erfassen eines Blutbildparameters von Blut in einem Blutgefäß. Das Verfahren umfasst das Einkoppeln 2501 eines ersten Sendesignals mit einer ersten Frequenz in das Blutgefäß, das Einkoppeln 2503 eines zweiten Sendesignals mit einer zweiten Frequenz in das Blutgefäß, das Empfangen 2505 eines ersten Empfangssignals bei der ersten Frequenz, das Empfangen 2507 eines zweiten Empfangssignals bei der zweiten Frequenz, das Ermitteln 2509 einer ersten Verlustgröße auf der Basis des ersten Sendesignals und des ersten Empfangssignals bei der ersten Frequenz, das Ermitteln 2511 einer zweiten Verlustgröße auf der Basis des zweiten Sendesignals und des zweiten Empfangssignals bei der zweiten Frequenz, das Bestimmen 2513 einer ersten Frequenzverschiebung der ersten Verlustgröße relativ zu einer ersten Referenzverlustgröße, das Bestimmen 2515 einer zweiten Frequenzverschiebung der zweiten Verlustgröße relativ zu einer zweiten Referenzverlustgröße, und das Bestimmen 2517 des Blutbildparameters auf der Basis der ersten Frequenzverschiebung und der zweiten Frequenzverschiebung.

Das in Fig. 25 dargestellte Verfahren kann beispielsweise durch die in Fig. 11 dargestellte Erfassungsvorrichtung ausgeführt werden.

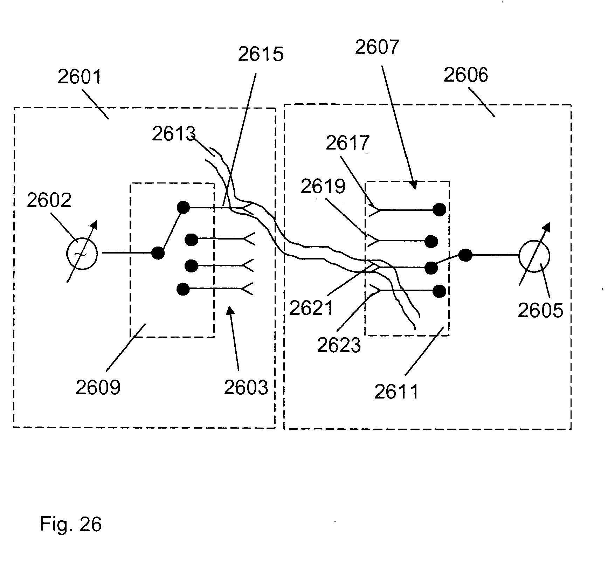

Fig. 26 zeigt eine Erfassungsvorrichtung mit einem Sender 2601, der beispielsweise einen durchstimmbaren Oszillator 2602 sowie eine Mehrzahl von Sendeantennen 2603 aufweist. Die Erfassungsvorrichtung umfasst ferner einen Verlustdetektor 2605, welcher beispielsweise einen Leistungsdetektor aufweisen kann. Ferner ist ein Empfänger 2606 mit einer Mehrzahl von Empfangsantennen 2607 vorgesehen.

Ein Ausgang des durchstimmbaren Oszillators 2602 ist beispielsweise mittels einer Schaltmatrix 2609 mit jedem Antenneneingang beispielsweise der Reihe nach oder in beliebiger Reihenfolge schaltbar verbindbar. In Analogie hierzu ist jeder Ausgang einer Empfangsantenne der Mehrzahl der Empfangsantennen 2607 mittels einer Schaltmatrix 2611 mit dem Verlustdetektor 2605 verbindbar.

Mittels der Schaltmatrix 2611 sowie der Schaltmatrix 2609 kann beispielsweise dasjenige Paar umfassend eine Sendeantenne und eine Empfangsantenne ausgewählt werden, das die beste Einkopplung eines Mikrowellensignals in ein in Fig. 26 schematisch dargestelltes Blutgefäß 2613 ermöglicht. Mittels der Schaltmatrizen 2609 und 2611 werden die Antennenpaar der Reihe nach ausgewählt beginnend beispielsweise mit einer ersten Sendeantenne 2615, mittels welcher ein Sendesignal ausgesendet wird. Die Schaltmatrizen 2609, 2611 können Schalter, beispielsweise Transistoren, aufweisen.

Empfangsseitig werden mittels der Schaltmatrix 2611 der Reihe nach die Empfangsantennen beginnend beispielsweise mit der Empfangsantenne 2617 zum Empfang eines entsprechenden Empfangssignals ausgewählt, wobei auf der Basis des Sendesignals und des Empfangssignals eine Verlustgröße erfasst wird. Im nächsten Schritt wird beispielsweise die Empfangsantenne 2619 ausgewählt, wobei wiederum auf der Basis des Sendesignals und eines durch die Empfangsantenne 2619 empfangenen Empfangssignals eine Verlustgröße mittels des Verlustdetektors erfasst wird. Danach wird beispielsweise die Empfangsantenne 2621 ausgewählt, wobei auf der Basis des Sendesignals und eines Empfangssignals eine weitere Verlustgröße erfasst wird. Im nächsten Schritt wird die Empfangsantenne 2623 ausgewählt und es wird auf der Basis des Sendesignals sowie eines durch die Empfangsantenne 2623 empfangenen Empfangssignals eine weitere Verlustgröße bestimmt. Im nächsten Schritt kann die Schaltmatrix 2609 beispielsweise eine weitere Sendeantenne auswählen, wobei die vorgenannten Schritte wiederholt werden können. Durch einen Vergleich der ermittelten Verlustgrößen wird beispielsweise die kleinste Verlustgröße ausgewählt. In dem in Fig. 26 dargestellten Beispiel ist beispielsweise zu erwarten, dass die Erfassungskonfiguration mit der Sendeantenne 2615 und der Empfangsantenne 2621 mit den geringsten Einkoppelverlusten behaftet ist, weil die Antennen 2615, 2621 unmittelbar oberhalb des Blutgefäßes liegen und somit eine optimale Einkopplung eines Signals in das Blutgefäß 2613 ermöglichen. Die ausgewählte Erfassungskonfiguration kann beispielsweise zur Erfassung eines Blutbildparameters herangezogen werden. Die vorstehend beschriebenen Auswahlschritte können in beliebiger Reihenfolge durchgeführt werden. So können beispielsweise für die Sendeantenne 2615 alle oder einige der Empfangsantennen 2607 getestet werden.

Die Sendeantennen 2603 bzw. die Empfangsantennen 2607 können sich hinsichtlich ihres Ortes und/oder hinsichtlich ihrer Feldkomponente, welche dominant angeregt werden soll, unterscheiden. Durch die Schaltmatrizen 2609 und 2611 ist dabei gewährleistet, dass für die jeweils gewählte Frequenz der optimale Erregertyp, beispielsweise Schleifenanenne, elektrische Dipolantenne, Patch-Antenne oder Erregerort ausgewählt werden kann.

Die in Fig. 26 dargestellte Erfassungsvorrichtung kann beispielsweise in einem aufpumpbaren Armband integriert sein. Zwischen den Erfassungen der Verlustgrößen, welche beispielsweise durch Messungen der Steuerparameter erfolgen können, kann Luft aus dem Armband abgelassen werden, so dass die Haut belüftet wird und sich kein Schweiß bildet. Ein Zeitabstand zwischen den Messungen kann dabei variabel erfolgen. Eine Messungen können beispielsweise in Abständen von 10 Minuten durchgeführt werden. Je nach Bedarf können jedoch häufigere Messungen durchgeführt werden, wobei die Häufigkeit der Messungen beispielsweise durch die Zeitpunkte der Einnahme der Mahlzeiten bestimmt sein kann.

Da insbesondere in den Pausen zwischen den Messungen die auf der Haut liegenden Sende- bzw. Empfangsantennen, welche jeweils durch eine Elektrodenplatte gebildet sein können, verrutschen können, kann durch die in Fig. 26 dargestellte Auswahl mehrerer Erreger sichergestellt werden, dass ein Erreger ausgewählt wird, welcher über dem Blutgefäß 2613 liegt. Mittels der jeweiligen Umschaltmatrix 2609 und 2611 kann somit derjenige Erreger ausgewählt werden, welcher ein Maximum einer Einkopplung von Mikrowellenenergie in das Blutgefäß 2613 ermöglicht.

**Patentansprüche**

1. Erfassungsvorrichtung (100) zum Erfassen eines Blutbildparameters eines Blutbestandteils von Blut in einem Blutgefäß (103), mit;
einem Sender (101), welcher ausgebildet ist, in das Blutgefäß ein erstes Sendesignal mit einer ersten Frequenz und ein zweites Sendesignal mit einer zweiten Frequenz einzukoppeln;
einem Empfänger (105), welcher ausgebildet ist, ein erstes Empfangssignal bei der ersten Frequenz und ein zweites Empfangssignal bei der zweiten Frequenz zu empfangen;
einem Verlustdetektor (107), welcher ausgebildet ist:

eine erste Verlustgröße auf der Basis des ersten Sendesignals und des ersten Empfangssignals zu ermitteln, und eine zweite Verlustgröße auf der Basis des zweiten Sendesignals und des zweiten Empfangssignals zu ermitteln, und

**dadurch gekennzeichnet dass** die Einrichtungsvorrichtung (100) weiter beinhaltet:

einen Prozessor (109), welcher ausgebildet ist, eine Relaxationszeitkonstante ($\tau$) des Blutbestandteils auf der Basis der folgenden Formel zu bestimmen:

$$\tau = \frac{1}{2\pi f_A}$$

wobei $f_A$ die Frequenz mit der größeren Verlustgröße bezeichnet.

2. Erfassungsvorrichtung nach Anspruch 1, wobei der Prozessor (109) ausgebildet ist, zumindest einen Blutbildparameter in Abhängigkeit der bestimmten Relaxationszeitkonstanten ($\tau$) zu ermitteln.

3. Erfassungsvorrichtung nach Anspruch 1 oder 2, wobei der Prozessor (109) ausgebildet ist, zumindest einen Blutbildparameter in Abhängigkeit der bestimmten Relaxationszeitkonstanten ($\tau$) mittels eines vorgegebenen Zusammenhangs zwischen der Konzentration des Blutbildparameters und der Relaxationszeitkonstanten ($\tau$) zu ermitteln.

4. Erfassungsvorrichtung nach einem der vorstehenden Ansprüche, wobei der zumindest eine Blutbildparameter eine Glucosekonzentration in dem Blut, eine Laktatkonzentration in dem Blut oder eine Sauerstoffkonzentration in dem Blut umfasst.

5. Erfassungsvorrichtung nach einem der vorstehenden Ansprüche, wobei der Verlustdetektor (107) ausgebildet ist, die erste Verlustgröße und die zweite Verlustgröße durch eine Zweitormessung zu bestimmen.

6. Erfassungsvorrichtung nach einem der vorstehenden Ansprüche, wobei der Verlustdetektor (107) einen Netzwerkanalysator oder einen Leistungsdetektor umfasst.

7. Erfassungsvorrichtung nach einem der vorstehenden Ansprüche, wobei der Verlustdetektor (107) ausgebildet ist, zur Bestimmung der ersten Verlustgröße und der zweiten Verlustgröße jeweils einen Vorwärtstransmissionsfaktor $S_{21}$ und einen Eingangsreflexionsfaktor $S_{11}$ zu bestimmen.

8. Erfassungsvorrichtung nach einem der vorstehenden Ansprüche, wobei der Verlustdetektor (107) ausgebildet ist, die erste Verlustgröße und die zweiten Verlustgröße jeweils auf der Basis der folgenden Formel zu bestimmen:

$$P_{Verlust} = 1 - \left| S_{11} \right|^2 - \left| S_{21} \right|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

9. Erfassungsvorrichtung nach einem der vorstehenden Ansprüche, wobei der Verlustdetektor (107) ausgebildet ist, die komplexe Dielektrizitätszahl (s") bei der jeweiligen Frequenz zur Bestimmung der jeweiligen Verlustgröße zu ermitteln.

10. Erfassungsvorrichtung nach einem der vorstehenden Ansprüche, wobei der Prozessor (109) ausgebildet ist, die Frequenz zu bestimmen, bei welcher der Imaginärteil der komplexen Dielektrizitätszahl (s") maximal ist, und die Relaxationszeitkonstante ($\tau$) in Abhängigkeit der bestimmten Frequenz zu ermitteln.

11. Erfassungsvorrichtung nach einem der vorstehenden Ansprüche, wobei der Sender (101) zum Einkoppeln des ersten Sendesignals oder des zweiten Sendesignals zumindest eine Sendeantenne, insbesondere eine Dipol-Antenne oder eine Rahmen-Antenne, umfasst, und wobei der Empfänger (105) zum Empfangen des ersten Empfangssignal und des zweiten Empfangssignals zumindest eine Empfangsantenne, insbesondere eine Dipol-Antenne, eine Rahmen-Antenne oder eine Patch-Antenne, welche von der Sendeantenne beabstandet ist, umfasst.

12. Erfassungsvorrichtung nach einem der vorstehenden Ansprüche, wobei der Sender (101) ausgebildet ist, das erste Sendesignal oder das zweite Sendesignal als eine transversal-elektrische (TE) Welle oder als eine transversal-magnetische (TM) Welle in das Blutgefäß (103) einzukoppeln, insbesondere longitudinal oder transversal bezüglich einer Blutflußrichtung einzukoppeln.

**13.** Erfassungsvorrichtung nach einem der vorstehenden Ansprüche, wobei der Sender (101) ausgebildet ist, das erste Sendesignal und das zweite Sendesignal sukzessive, insbesondere mittels eines durchstimmbaren Oszillators, oder gleichzeitig, insbesondere mittels das erste Sendesignal und das zweite Sendesignal umfassenden Breitbandsignals, in das Blutgefäß (103) einzukoppeln.

**14.** Verfahren zum Erfassen eines Blutbildparameters eines Blutbestandteils von Blut in einem Blutgefäß, mit:

Einkoppeln (501) eines ersten Sendesignals mit einer ersten Frequenz und eines zweites Sendesignals mit einer zweiten Frequenz in das Blutgefäß;
Empfangen (503) eines ersten Empfangssignals bei der ersten Frequenz und eines zweiten Empfangssignal bei der zweiten Frequenz;
Ermitteln (505) einer ersten Verlustgröße auf der Basis des ersten Sendesignals und des ersten Empfangssignals;
Ermitteln (507) einer zweiten Verlustgröße auf der Basis des zweiten Sendesignals und des zweiten Empfangssignals, und
Bestimmen (509) einer Relaxationszeitkonstanten ($\tau$) des Blutbestandteils auf der Basis der folgenden Formel:

$$\tau = \frac{1}{2\pi f_A}$$

wobei $f_A$ die Frequenz mit der größeren Verlustgröße bezeichnet.

## Claims

**1.** Detection device (100) for detecting a blood picture parameter of a blood constituent of blood in a blood vessel (103), comprising:

a transmitter (101), which is configured to couple a first transmission signal with a first frequency and a second transmission signal with a second frequency into the blood vessel;
a receiver (105), which is configured to receive a first reception signal at the first frequency and a second reception signal at the second frequency;
a loss detector (107), which is configured to:

determine a first loss variable on the basis of the first transmission signal and the first reception signal, and determine a second loss variable on the basis of the second transmission signal and the second reception signal; and
**characterized in that** the detection device (100) further comprises:

a processor (109), which is configured to determine a relaxation time constant ($\tau$) of the blood constituent on the basis of the following formula:

$$\tau = \frac{1}{2\pi f_A}$$

where $f_A$ denotes the frequency with the greater loss variable.

**2.** Detection device according to Claim 1, wherein the processor (109) is configured to determine at least one blood picture parameter depending on the determined relaxation time constant ($\tau$).

**3.** Detection device according to Claim 1 or 2, wherein the processor (109) is configured to determine at least one blood picture parameter depending on the determined relaxation time constant ($\tau$) by means of a predetermined relationship between the concentration of the blood picture parameter and the relaxation time constant ($\tau$).

4. Detection device according to one of the preceding claims, wherein the at least one blood picture parameter comprises a glucose concentration in the blood, a lactate concentration in the blood or an oxygen concentration in the blood.

5. Detection device according to one of the preceding claims, wherein the loss detector (107) is configured to determine the first loss variable and the second loss variable by means of a two-port measurement.

6. Detection device according to one of the preceding claims, wherein the loss detector (107) comprises a network analyzer or a power detector.

7. Detection device according to one of the preceding claims, wherein the loss detector (107) is configured to determine in each case a forward transmission factor $S_{21}$ and an input reflection factor $S_{11}$ in order to determine the first loss variable and the second loss variable.

8. Detection device according to one of the preceding claims, wherein the loss detector (107) is configured to determine in each case the first loss variable and the second loss variable on the basis of the following formula:

$$P_{loss} = 1 - |S_{11}|^2 - |S_{21}|^2,$$

where $P_{loss}$ denotes the respective loss variable, and where $S_{11}$ denotes the input reflection factor and $S_{21}$ denotes the forward transmission factor.

9. Detection device according to one of the preceding claims, wherein the loss detector (107) is configured to determine the complex permittivity ($\varepsilon''$) at the respective frequency for determining the respective loss variable.

10. Detection device according to one of the preceding claims, wherein the processor (109) is configured to determine the frequency at which the imaginary part of the complex permittivity ($\varepsilon''$) is at a maximum and to determine the relaxation time constant ($\tau$) depending on the determined frequency.

11. Detection device according to one of the preceding claims, wherein the transmitter (101) for coupling-in the first transmission signal or the second transmission signal comprises at least one transmission antenna, in particular a dipole antenna or a frame antenna, and wherein the receiver (105) for receiving the first reception signal and the second reception signal comprises at least one reception antenna, in particular a dipole antenna, a frame antenna or a patch antenna, which is at a distance from the transmission antenna.

12. Detection device according to one of the preceding claims, wherein the transmitter (101) is configured to couple the first transmission signal or the second transmission signal into the blood vessel (103) as a transverse electric (TE) wave or as a transverse magnetic (TM) wave, in particular longitudinally or transversely with respect to a blood flow direction.

13. Detection device according to one of the preceding claims, wherein the transmitter (101) is configured to couple the first transmission signal and the second transmission signal into the blood vessel (103) successively, in particular by means of a tunable oscillator, or simultaneously, in particular by means of a broadband signal comprising the first transmission signal and the second transmission signal.

14. Method for detecting a blood picture parameter of a blood constituent of blood in a blood vessel, comprising the following steps:

coupling (501) a first transmission signal with a first frequency and a second transmission signal with a second frequency into the blood vessel;
receiving (503) a first reception signal at the first frequency and a second reception signal at the second frequency;
determining (505) a first loss variable on the basis of the first transmission signal and the first reception signal;
determining (507) a second loss variable on the basis of the second transmission signal and the second reception signal; and
determining (509) a relaxation time constant ($\tau$) of the blood constituent on the basis of the following formula:

$$\tau = \frac{1}{2\pi f_A}$$

where $f_A$ denotes the frequency with the greater loss variable.

**Revendications**

1. Dispositif d'acquisition (100) pour acquérir un paramètre de formule sanguine d'un composant sanguin du sang dans un vaisseau sanguin (103), comprenant :

   un émetteur (101) qui est configuré pour injecter dans le vaisseau sanguin un premier signal émis ayant une première fréquence et un deuxième signal émis ayant une deuxième fréquence ;
   un récepteur (105) qui est configuré pour recevoir un premier signal reçu à la première fréquence et un deuxième signal reçu à la deuxième fréquence ;
   un détecteur de pertes (107) qui est configuré pour :

   détecter une première grandeur de perte en se basant sur le premier signal émis et le premier signal reçu ; et détecter une deuxième grandeur de perte en se basant sur le deuxième signal émis et le deuxième signal reçu ; et
   **caractérisé en ce que** le dispositif d'acquisition (100) comprend en outre :

   un processeur (109) qui est configuré pour déterminer une constante de temps de relaxation ($\tau$) du composant sanguin en se basant sur la formule suivante :

$$\tau - \frac{1}{2\pi f_A}$$

   $f_A$ désignant la fréquence avec la plus grande grandeur de perte.

2. Dispositif d'acquisition selon la revendication 1, avec lequel le processeur (109) est configuré pour détecter au moins un paramètre de formule sanguine en fonction de la constante de temps de relaxation ($\tau$) déterminée.

3. Dispositif d'acquisition selon la revendication 1 ou 2, avec lequel le processeur (109) est configuré pour détecter au moins un paramètre de formule sanguine en fonction de la constante de temps de relaxation ($\tau$) déterminée au moyen d'une relation prédéfinie entre la concentration du paramètre de formule sanguine et la constante de temps de relaxation ($\tau$).

4. Dispositif d'acquisition selon l'une des revendications précédentes, avec lequel l'au moins un paramètre de formule sanguine comprend une concentration du glucose dans le sang, une concentration du lactate dans le sang, ou une concentration de l'oxygène dans le sang.

5. Dispositif d'acquisition selon l'une des revendications précédentes, avec lequel le détecteur de pertes (107) est configuré pour déterminer la première grandeur de perte et la deuxième grandeur de perte par le biais d'une mesure à deux portes.

6. Dispositif d'acquisition selon l'une des revendications précédentes, avec lequel le détecteur de pertes (107) comprend un analyseur de réseau ou un détecteur de puissance.

7. Dispositif d'acquisition selon l'une des revendications précédentes, avec lequel le détecteur de pertes (107) est configuré, en vue de la détermination de la première grandeur de perte et de la deuxième grandeur de perte, pour déterminer à chaque fois un facteur de transmission dans le sens direct $S_{21}$ et un facteur de réflexion d'entrée $S_{11}$.

**8.** Dispositif d'acquisition selon l'une des revendications précédentes, avec lequel le détecteur de pertes (107) est configuré pour déterminer la première grandeur de perte et la deuxième grandeur de perte à chaque fois en se basant sur la formule suivante :

$$P_{Perte} = 1 - |S_{11}|^2 - |S_{21}|^2,$$

$P_{Perte}$ désignant la grandeur de perte correspondante, $S_{11}$ le facteur de réflexion d'entrée et $S_{21}$ le facteur de transmission dans le sens direct.

**9.** Dispositif d'acquisition selon l'une des revendications précédentes, avec lequel le détecteur de pertes (107) est configuré pour détecter l'indice diélectrique complexe ($\varepsilon''$) à la fréquence correspondante afin de déterminer la grandeur de perte correspondante.

**10.** Dispositif d'acquisition selon l'une des revendications précédentes, avec lequel le processeur (109) est configuré pour déterminer la fréquence à laquelle la partie imaginaire de l'indice diélectrique complexe ($\varepsilon''$) est maximale et pour détecter la constante de temps de relaxation ($\tau$) en fonction de la fréquence déterminée.

**11.** Dispositif d'acquisition selon l'une des revendications précédentes, avec lequel l'émetteur (101), pour l'injection du premier signal émis ou du deuxième signal émis, comprend au moins une antenne d'émission, notamment une antenne dipôle ou une antenne cadre, et avec lequel le récepteur (105), pour la réception du premier signal reçu et du deuxième signal reçu, comprend au moins une antenne de réception, notamment une antenne dipôle, une antenne cadre ou une antenne composite, qui est éloignée de l'antenne d'émission.

**12.** Dispositif d'acquisition selon l'une des revendications précédentes, avec lequel l'émetteur (101) est configuré pour injecter dans le vaisseau sanguin (103) le premier signal émis ou le deuxième signal émis sous la forme d'une onde électrique transversale (TE) ou sous la forme d'une onde magnétique transversale (TM), notamment pour l'injecter longitudinalement ou transversalement par rapport à un sens de circulation du sang.

**13.** Dispositif d'acquisition selon l'une des revendications précédentes, avec lequel l'émetteur (101) est configuré pour injecter successivement dans le vaisseau sanguin (103) le premier signal émis et le deuxième signal émis, notamment au moyen d'un oscillateur accordable, ou simultanément, notamment au moyen d'un signal à large bande qui englobe le premier signal émis et le deuxième signal émis.

**14.** Procédé d'acquisition d'un paramètre de formule sanguine d'un composant sanguin du sang dans un vaisseau sanguin, comprenant :

injection (501) dans le vaisseau sanguin d'un premier signal émis ayant une première fréquence et d'un deuxième signal émis ayant une deuxième fréquence ;
réception (503) d'un premier signal reçu à la première fréquence et d'un deuxième signal reçu à la deuxième fréquence ;
détection (505) d'une première grandeur de perte en se basant sur le premier signal émis et le premier signal reçu ;
détection (507) d'une deuxième grandeur de perte en se basant sur le deuxième signal émis et le deuxième signal reçu ; et
détermination (509) d'une constante de temps de relaxation ($\tau$) du composant sanguin en se basant sur la formule suivante :

$$\tau = \frac{1}{2\pi f_A}$$

$f_A$ désignant la fréquence avec la plus grande grandeur de perte.

Fig. 1

Fig. 2

Fig. 3

Fig. 4

Einkoppeln eines ersten Sendesignals — 501

Einkoppeln eines zweiten Sendesignals — 503

Ermitteln einer ersten Verlustgröße — 505

Ermitteln einer zweiten Verlustgröße — 507

Bestimmen einer Relaxationszeitkonstante — 509

Fig. 5

Fig. 6

Fig. 7

Fig. 8

Fig. 9

Fig. 10

1103

Sender

Empfänger

1101

1105

Verlustdetektor

1107

Speicher

Prozessor

1111

1109

1100

Fig. 11

Fig. 12A

Fig. 12B

Fig. 12C

1301

1303

1307

Blutgefäß

$E_{tangential}$

1305

1308

Fig. 13A

1309

1307

Blutgefäß

$H_{tangential}$

1310

Fig. 13B

1313    1315
1311                        1307

Blutgefäß

$E_{longitudinal}$

1317

Fig. 13C

1307

1319

Blutgefäß

$H_{longitudinal}$

1320

Fig. 13D

1407  1408  1401  1403  1405

Fig. 14A

1409  1413  1411  1415

Fig. 14B

Fig. 15A

Fig. 15B

Fig. 16A

Fig. 16B

Fig. 17

Fig. 18

Fig. 19

Fig. 20

2103   2105

2101   2106

2109

Fig. 21

−27.58

−27.6

−27.62

−27.64

−27.66

−27.68

−27.7

$|S_{21}|$ in dB

2203

2201

6.2   6.25   6.3   6.35   6.4   6.45   6.5   6.55

f in GHz

Fig. 22

Fig. 23

| Durchm. El | A | B | C | D | E | F | G |
|---|---|---|---|---|---|---|---|
| 6 mm | <1 | +2 | -1 | -9 | -17 | +11 | <1 |
| 3,4 mm | <1 | +4 | -1 | -13 | - | - | -10 |

Fig. 24

2501
Einkoppeln
Einkoppeln
2503

2505
Empfangen
Empfangen
2507

2509
Ermitteln einer
Verlustgröße
Ermitteln einer
Verlustgröße
2511

2513
Bestimmen einer
Frequenzverschiebung
Bestimmen einer
Frequenzverschiebung
2515

Bestimmen des
Blutbildparameters
2517

Fig. 25

Fig. 26

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2009027814 A1

**In der Beschreibung aufgeführte Nicht-Patentliteratur**

- **ANDREAS CADUFF et al.** Non-invasive glucose monitoring in patients with Type 1 diabetes: A multi-sensor system combining sensors for dielectric and optical characterization of skin. *Biosensors and Bioelectronics,* 2009, vol. 24, 2778-2784
- **BUFORD RANDAL JEAN et al.** A microwave frequency sensor for non-invasive blood-glucose measurement. *SAS 2008 - IEEE Sensors Applications Symposium, Atlanta, GA,* 12. Februar 2008
- **M. MCCLUNG.** Calibration methodology for a microwave non-invasive glucose sensor. *Master Thesis,* Mai 2008
- **NETTER, F.N.** Atlas der Anatomie. Thieme-Verlag, 2006